# EUROPEAN PATENT SPECIFICATION

(11) **EP 2 453 889 B1**
(45) Date of publication and mention of the grant of the patent: **08.04.2015**
(21) Application number: 10734105.9
(22) Date of filing: 14.07.2010
(51) Int. Cl.: A61K 31/353, A61K 36/185, A61K 36/70, A61K 36/45, A61K 31/04, A61K 31/02, A61P 1/02

(54) **USE OF PROANTHOCYANIDINS FOR PRODUCTION OF AN ANTIADHESIVE PREPARATION**
VERWENDUNG VON PROANTHOCYANIDINEN FÜR DIE HERSTELLUNG EINER ANTIHAFTZUBEREITUNG
UTILISATION DE PROANTHOCYANIDINES POUR LA PRODUCTION D'UNE PRÉPARATION ANTI-ADHÉSIVE

(30) Priority: 14.07.2009 DE 102009027696
(43) Date of publication of application: 23.05.2012
(73) Proprietor: Westfälische Wilhelms-Universität Münster, 48149 Münster (DE)
(72) Inventor: HENSEL, Andreas, 48161 Münster (DE); LECHTENBERG, Matthias, 48161 Münster (DE); PETEREIT, Frank, 48329 Havixbeck (DE); LÖHR, Gesine, 45478 Mülheim (DE); BEIKLER, Thomas, 40225 Düsseldorf (DE); LOUIS, Andrea, 59302 Oelde (DE)
(74) Representative: Michalski Hüttermann & Partner Patentanwälte mbB
(86) International application number: PCT/EP2010/060161
(87) International publication number: WO 2011/006938

(56) References cited:
- DE-B3-102005 053 926
- US-A1- 2007 134 193
- US-A1- 2008 306 141
- ANKE J ET AL: "Isolation, structure elucidation, cytotoxicity and anti-adhesive properties of proanthocyanidins from Rumex acetosa L", PLANTA MEDICA, vol. 73, no. 9, August 2007 (2007-08), page 924, XP9139121, & 55TH ANNUAL CONGRESS OF THE SOCIETY-FOR-MEDICINAL-PLANT-RESEARCH; GRAZ, AUSTRIA; SEPTEMBER 02 -06, 2007 ISSN: 0032-0943
- LENGSFELD C ET AL: "Antiadhesive natural products for a new cytoprotective strategy against the early stages of infection by pathogens", PLANTA MEDICA, THIEME VERLAG, DE, vol. 75, no. 9, 1 July 2009 (2009-07-01), page 888, XP009139183, ISSN: 0032-0943
- LOEHR G ET AL: "Antiadhesive effects of natural extracts out of Myrothamnus flabellifolia Welw. and Rumex acetosa L. against Porphyromonas gingivalis", PLANTA MEDICA, THIEME VERLAG, DE, vol. 75, no. 9, 1 July 2009 (2009-07-01), pages 897-898, XP009139182, ISSN: 0032-0943
- GESCHER K ET AL: "Potent antiviral effect of a polyphenol-enriched Rumex acetosa L. extract against herpes simplex virus type 1 via interaction with viral envelope proteins", CYTOMETRY, ALAN LISS, NEW YORK, US, vol. 75, no. 9, 1 July 2009 (2009-07-01), page 884, XP009139120, ISSN: 0196-4763
- DATABASE WPI Week 199122 Thomson Scientific, London, GB; AN 1991-159326 XP002602926, & JP 3 093723 A (WATADA Y) 18 April 1991 (1991-04-18)
- ANKE J ET AL: "Proanthocyanidins and phenolglycosides from Rumex acetosa L", PLANTA MEDICA, THIEME VERLAG, DE, vol. 72, no. 11, 1 September 2006 (2006-09-01), page 995, XP009139118, ISSN: 0032-0943
- DATABASE WPI Week 198441 Thomson Scientific, London, GB; AN 1984-253069 XP002602927, & JP 59 152311 A (SUNSTAR KK) 31 August 1984 (1984-08-31)
- ANKE JENNIFER ET AL: "Procyanidins from Myrothamnus flabellifolia.", NATURAL PRODUCT RESEARCH 2008 LNKD- PUBMED:18932087, vol. 22, no. 14, 2008, pages 1237-1248, XP9139130, ISSN: 1478-6427
- LOEHR G ET AL: "Effects of extracts from Myrothamnus flabellifolia Welw. on Streptococcus mutans induced biofilm formation and Porphyromonas gingivalis induced inflammation parameters in KB cells", PLANTA MEDICA, vol. 75, no. 9, 1 July 2009 (2009-07-01), page 1051, XP9139180, & 57TH INTERNATIONAL CONGRESS AND ANNUAL MEETING OF THE SOCIETY-FOR-MEDICINAL-PLANT-RESEARCH-AND-N ATUR; GENEVA, SWITZERLAND; AUGUST 16 -20, 2009 ISSN: 0032-0943
- PETEREIT F ET AL: "Proanthocyanidins from the herb of Myrothamnus flabellifolia Welw", PLANTA MEDICA, vol. 72, no. 11, September 2006 (2006-09), page 1022, XP9139122, & 54TH ANNUAL CONGRESS ON MEDICINAL PLANT RESEARCH; HELSINKI, FINLAND; AUGUST 29 -SEPTEMBER 02, 2006 ISSN: 0032-0943
- ANKE J ET AL: "Procyanidins from Myrothamnus flabellifolia", MEDICINAL & AROMATIC PLANTS ABSTRACTS, SCIENTIFIC PUBLISHERS, SCIENTIFIC PUBLISHERS, NEW DELHI - INDIA, vol. 31, no. 5, 20 September 2008 (2008-09-20), XP018027332, ISSN: 0250-4367

### SEQUENCE LISTING

<110> Westphalian Wilhelm University Münster
<120> Use of proanthocyanidins for production of an antiadhesive preparation
<130> UD40364
<160> 12
<170> PatentIn version 3.5
<210> 1
   <211> 19
   <212> DNA
   <213> Artificial Sequence
<220>
   <223> 16S-rRNA Forward
<400> 1
   taacagtttt cgctgagga 19
<210> 2
   <211> 22
   <212> DNA
   <213> Artificial Sequence
<220>
   <223> 16S-rRNA reverse
<400> 2
   tgtaagggcc gtgttgaatt ga 22
<210> 3
   <211> 16
   <212> DNA
   <213> Artificial Sequence
<220>
   <223> 16S-rRNA Probe
<400> 3
   gtgaggaagg tgtgga 16
<210> 4
   <211> 18
   <212> DNA
   <213> Artificial Sequence
<220>
   <223> kpg forward
<400> 4
   gtgtatgttg ggctatct 18
<210> 5
   <211> 19
   <212> DNA
   <213> Artificial Sequence
<220>
   <223> kpg reverse
<400> 5
   tccttactgc caccccttt 19
<210> 6
   <211> 16
   <212> DNA
   <213> Artificial Sequence
<220>
   <223> kpg probe
<400> 6
   tggagggaag aggagt 16
<210> 7
   <211> 20
   <212> DNA
   <213> Artificial Sequence
<220>
   <223> rpgA forward
<400> 7
   gcaggatgag atgaacgaaa 20
<210> 8
   <211> 16
   <212> DNA
   <213> Artificial Sequence
<220>
   <223> rpgA reverse
<400> 8
   gtgaccaccg aaagta 16
<210> 9
   <211> 16
   <212> DNA
   <213> Artificial Sequence
<220>
   <223> rpgA, Probe
<400> 9
   aaacacccga acaaca 16
<210> 10
   <211> 18
   <212> DNA
   <213> Artificial Sequence
<220>
   <223> fimA forward
<400> 10
   acgaataacc cagagaat 18
<210> 11
   <211> 18
   <212> DNA
   <213> Artificial Sequence
<220>
   <223> fimA reverse
<400> 11
   ctgaccaacg agaaccca 18
<210> 12
   <211> 17
   <212> DNA
   <213> Artificial Sequence
<220>
   <223> fimA probe
<400> 12
   tcacagagtc tgctcac 17

## Description

The invention relates to the use of proanthocyanidins for the production of a preparation that prevents the adhesion of microorganisms on body surfaces.

*Porphyromonas gingivalis* (Pg) is a gram-negative, facultatively anaerobic bacterium, which is known to be the main cause of periodontitis and gingivitis, which are diseases of the periodontium, occurring in about 30% of all people in Central Europe. Furthermore, systemic infection with Pg is a risk factor for infarction.

Treatment strategies against Pg-induced disease of the oral cavity, which starts with infection of the oral mucosa and of the gingiva in the region of the periodontium, continues with lysis of the gingiva and ultimately leads to tooth loss, are still conservatively symptomatic and surgical. The economic losses from these diseases are immense. Specific treatment strategies, primarily with prophylactic and cytoprotective action, appear to us to be unavailable at present.

The strategy of Pg, a normal inhabitant of the human oral flora, for aggressive and virulent attack of the gingiva has as a pre-condition the adhesion of the microbe to epithelial cells of the oral mucosa, namely the buccal mucosa and peridontium. This adhesion is provided by various bacterial adhesins of Pg, which are located *inter alia* on fimbriae of the bacterium. The most important relevant adhesins are on the one hand hemagglutinins, and on the other hand gingipains (Lys- and Arg-gingipain). These adhere preferentially to protein structures of the target cell. Once adhesional contact is made, another domain of the gingipains becomes active, which in its turn has protease activity. In this way the bacterium infiltrates the dental epithelium proteolytically, mobilizes calcium from the tooth itself and promotes the deterioration of the periodontium proteolytically. Other additional virulence factors are activated and lead to further destruction of the tissue. If capillary vessels are attacked proteolytically, there may in addition be systemic invasion of the blood compartment by the pathogen. There is then a risk that the bacterial hemagglutinin may cause aggregation of erythrocytes, which can then lead to formation of thrombi, with a greatly increased risk of ischemic tissue damage.

Epidemiological studies have in fact established a link between periodontitis and increased incidence of infarctions.

Conclusive preventive strategies for early inhibition of periodontitis are still not feasible. Oral hygiene products with antibacterial activity could serve to that purpose, but due to the low specificity of those products, both the physiology of the oral microflora can change over the time and host tissues can be harmed by long term use of such biocide products.

Much the same applies to another bacterium occurring in the oral cavity, namely the gram-positive, facultatively anaerobic bacterium *Streptococcus mutans,* which is regarded as the indicator organism of dental caries, and is in particular able to form resistant biofilms, for which once again adhesins are responsible. These biofilms can in their turn harbour Pg-microbes.

In DE 102005053926 the use of an antibacterial agent from mixture of plant drugs, comprises Rumex acetosa L.-family, Rumicisherba, Verbena officinalis, Sambucus nigra, Primulaveris and Gentianalutea, e.g. to treat infection on topical skin and mucous membrane is described.

The object of the present invention is therefore to provide a preparation that prevents the adhesion of at least one microorganism on a biological or biocompatible surface.

This object is achieved with the features of the main claim. The subclaims present preferred embodiments.

### Description of the invention

The invention as characterized in the claims relates to the use of a preparation containing at least one proanthocyanidin from a plant *Rumex acetosa,* for the production of a preparation that prevents the bacterial adhesion of at least one microorganism on a biological or biocompatible surface. In particular, the invention relates to a composition containing at least one oligomeric or polymeric flavan-3-ol unit (proanthocyanidin) from a plant *Rumexacetosa* extract, for use in prevention or treatment of gingivitis, periodontitis, caries, ulcer of the oral mucosa (aphtha), or gingivostomatitis wherein the composition prevents the adhesion of at least one bacterium on mucosa, tongue, palate, gingiva, gingival pocket or tooth surface in a exposure time that does not exceed 10 minutes.

Proanthocyanidins can occur in various degrees of oligomerization, very often as dimers or trimers, but also in higher oligomeric or polymeric flavan-3-ol compounds, which can be assigned to the A, B and C series, depending on the type of linkage of the interflavan bond (4⇒6 or 4⇒8 or double-bonded with an ether bridge).

An example of monomeric flavan-3-ol (epicatechol) is shown in the following formula:

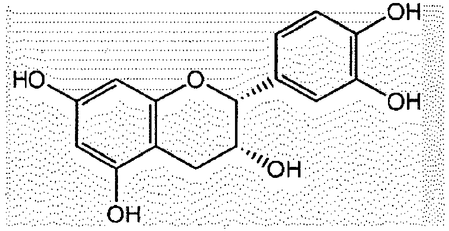

Other frequently occurring monomers are catechol, afzelechin, epiafzelechin, gallocatechol or epigallocatechin.
Proanthocyanidins can undergo secondary modification biosynthetically, e.g. through esterification with aromatic acids, e.g. gallic acid or p-hydroxy-benzoic acid.

Within the scope of the present invention, it was found for the first time, surprisingly, that proanthocyanidins that are contained in plant extracts of *Rumex acetosa* as claimed as well as *Rhododendron ferrugineum* and/or *Myrothamnus flabellifolia,* and preparations therefrom, are in addition able to prevent the adhesion of microorganisms on body surfaces.

*Rumex acetosa,* sorrel, is a plant species that belongs to the knotgrass family (Polygonaceae). *Rhododendron ferrugineum,* the russet-leaved rhododendron, is a plant species from the Rhododendron genus and belongs to the heather family (Ericaceae). The plant *Myrothamnus flabellifolia,* a resurrection plant, belongs to the only plant genus of the family of Myrothamnaceae in the small order of the gunnera. Its natural habitat is arid southern Africa.

It was found in particular that the aforementioned extracts exert a very pronounced antiadhesive action against the bacterial attachment process of various bacteria to body surfaces, in particular to human epithelial cells of the buccal mucosa. Investigation of these extracts showed, among other things, a high content of monomeric flavan-3-ols, and oligomeric and polymeric proanthocyanidins, in particular from the subgroup of the procyanidins. As well as these extracts, such effects are also suggested and demonstrated with isolated oligomeric proanthocyanidins.

The cornerstone of the invention is the fact that successful suppression of bacterial adhesion on a body surface can lead to a decrease in infection of the tissue in question. As a result, for example the symptoms of periodontitis and gingivitis can be reduced considerably.

It should be pointed out that the observed antiadhesive effects already occur at concentrations and/or times of action for which no antibacterial or even cytotoxic effects yet occur.

Moreover cytoprotective effects especially of *Myrothamnus flabellifolia* extracts against inflammation related parameters of human cells were shown.

Furthermore, it has been demonstrated that the test extracts have an influence on the formation of a microbially produced biofilm. Reference is made to an example.

It is preferably envisaged that the composition according to the invention has at least one plant extract from one of the aforementioned plants. Said extract can preferably be an aqueous extract, an ethanolic extract, an acetone extract and/or an ethyl acetate extract. The extract is preferably in the form of a dry extract, before it is used for production of the preparation according to the invention.

Furthermore, it is preferably envisaged according to the invention that the composition has at least one isolated and/or enriched and/or chemically modified proanthocyanidin.

The biological or biocompatible surface is preferably at least one surface selected from the group comprising a human or animal body surface, surface of an implant, of a prosthesis, of an epithesis, of a catheter, of a drainage tube, of a wound care article, of a hygiene article, of a contact lens and/or of a hearing aid.

Preferably the preparation according to the invention (dosage form) comprises oral hygiene products such as mouth wash, a tincture, an ointment, a toothpaste, a lozenge, a gel, impregnated inserts, rapid-release and dissolving granules, powders or other solid dosage forms, chewing gums or other plastic or elastic masses, impregnated tooth cleaning aids, such as dental floss, toothbrushes and/or a rinse. The aforesaid wound care article is preferably a dressing. The aforesaid hygiene article is preferably a diaper and/or an incontinence insert.

The aforementioned body surface is preferably at least one surface selected from the group comprising skin, mucosa, tongue, palate, gingiva, gingival pocket and/or tooth surface.

It is further envisaged that the preparation according to the invention is a preparation for preventing the formation of and/or for dissolving bacterial coatings, biofilms and/or plaque.

Basically the term "biofilm" is a general term for all bacterial coatings, including the plaque known from dentistry. A layer of varying thickness is formed, which is stabilized in particular by bacterial polymers and adhesins. This layer offers bacterial organisms on the one hand protection against antibacterial measures, such as antibiotics or abrasive agents (such as toothpaste), and on the other hand it also creates microenvironments, promoting the growth of certain bacterial organisms. For example, a partially oxygen-free microenvironment may be created, which promotes the growth of obligate or facultatively anaerobic bacteria, e.g. *P. gingivalis* or *S. mutans.*

The preparation according to the invention is able to prevent the formation of such coatings or even dissolve them.

Moreover, it is preferably envisaged that the preparation according to the invention is a preparation for the prevention and/or treatment of bacterially induced oral diseases and/or bacterially induced infarctions or the precursors thereof.

The term "bacterially induced oral diseases" means hereinafter diseases such as periodontitis, gingivitis, caries, ulcers of the oral mucosae (aphthae), and gingivostomatitis.

The term "bacterially induced infarctions or precursors thereof" means hereinafter diseases such as angina pectoris, coronary heart disease, myocardial infarction, stroke, pulmonary embolism and/or ischemic optic neuropathy. These diseases may be caused by bacterial hemagglutinin, which then - for example after proteolytic invasion of *P. gingivalis* pathogens into the dental epithelium of the gingiva - can lead to aggregation of erythrocytes, which in its turn can lead to the thrombogenesis.

Preferably the preparation according to the invention (dosage form) comprises oral hygiene products such as mouth wash, a tincture, an ointment, a toothpaste, a lozenge, a gel, impregnated inserts, rapid-releasing and dissolving granules, powder or other solid dosage forms, chewing gums or other plastic or elastic masses, impregnated tooth cleaning aids, such as dental floss, toothbrushes and/or a rinse.

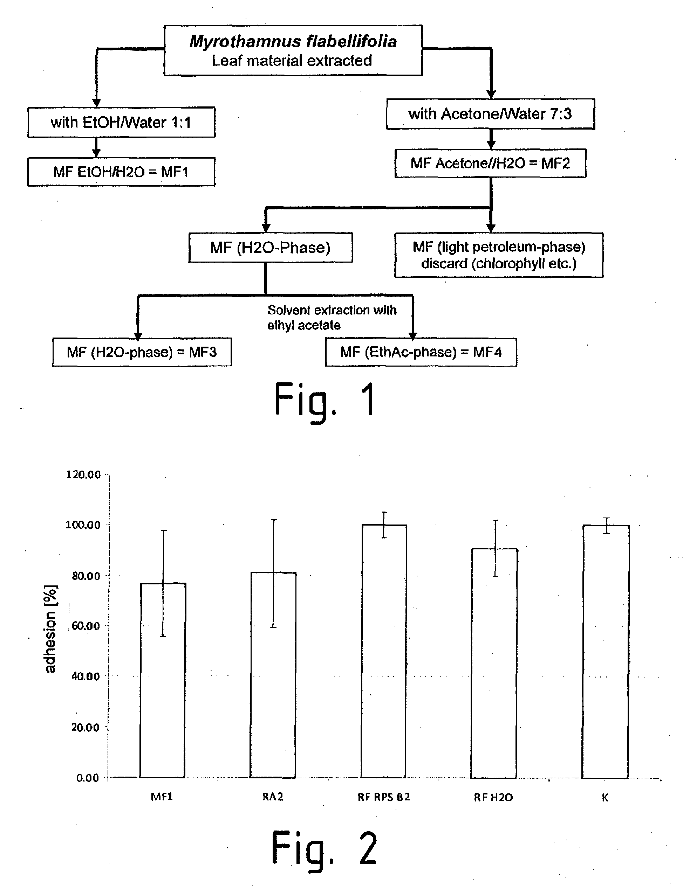

Fig. 1 shows an extraction scheme for *Myrothamnus flabelifola* (MF), which also applies similarly to *Rumex acetosa* (RA). It should be noted that the fraction MF1/RA1 is a fraction extracted with ethanol/water 1:1. The fraction MF2/RA2 was instead extracted with an acetone-water mixture. In some cases the aqueous supernatant was shaken again with ethyl acetate. In this way it was possible to enrich the aqueous phase (MF3/RA3) with higher-molecular oligomeric proanthocyanidins (≥ DP 4, tetramers), whereas in the ethyl acetate phase (MF4/RA4) there was preferential enrichment of low-molecular oligomeric proanthocyanidins (≤ DP 4). An alternative extraction scheme for extracts from *Rhododendron ferrugineum* is described below, the corresponding extract being designated as RF RPS B2.

Basically at least one plant extract can be obtained by extraction in water or an ethanol/water mixture. Preferably, however, it is envisaged that at least one plant extract was obtained by extraction comprising the following steps:
a) extraction in an acetone-water mixture,
b) obtaining the aqueous phase,
c) shaking the aqueous phase with ethyl acetate, and
d) obtaining the aqueous phase.

The extracts obtained can basically be used in liquid - i.e. dissolved - form. Preferably, however, it is envisaged that these are used in dried form. For this, the extracts obtained are preferably centrifuged and/or then freeze-dried.

It was then found, surprisingly, that the higher-molecular fractions have lower toxicity or antibacterial action, but conversely they displayed better antiadhesive action. The reason for this previously unknown fact is still unclear; there are, however, indications that the higher-molecular proanthocyanidin fractions are taken up less well by the cells than the low-molecular fractions, so have no or slight intracellular bioavailability and therefore act preferentially at the surfaces of the cells.

It is also preferably envisaged that at least one proanthocyanidin is a procyanidin. Procyanidins are proanthocyanidins that are constructed only from catechol or epicatechol units, since these procyanidins have practically no direct cytotoxicity in the field of application according to the invention.

It is also envisaged according to the invention that the composition is used at a concentration that does not have antimicrobial and/or bactericidal action in an exposure time that is usually employed.

Basically it is known that oligomeric proanthocyanidins in particular have antioxidant effects. Thus, it was shown in laboratory tests that the antioxidant action of certain proanthocyanidins is 20 times stronger than vitamin C and 50 times stronger than vitamin E (Shi et al. 2003). It is also reported that at high enough dosages or exposure times, proanthocyanidins can have antibacterial action.

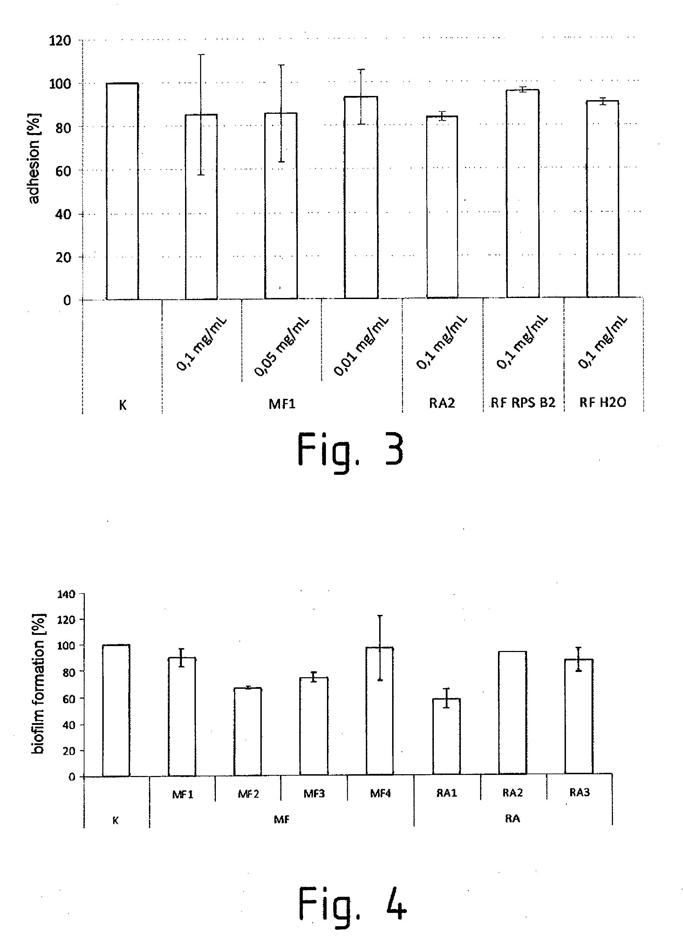

The present invention, however, expressly relates neither to antioxidant nor to antibacterial and/or bactericidal action; rather it relates exclusively to a decrease in adhesion. In this respect, according to the inventors' findings, surprisingly, very much lower concentrations or exposure times are required than are necessary for an antibacterial action. Thus, antiadhesive effects are already achieved with concentrations of less than 100 µg/ml. It was shown, for example, that the adhesion of *P. gingivalis* to KB cells, on incubation with various extracts according to the invention (in particular RA2 and MF1) at a concentration of 50 µg/ml, was already reduced significantly in an incubation time of 90 min (Fig. 3).

Basically there is a functional relationship both between exposure time and antibacterial action on the one hand and between concentration and antibacterial action on the other hand; it is not, however, proportional, because with increasing exposure time or concentration the antibacterial action reaches saturation.

This is relevant in particular because an antibacterial and/or bactericidal concentration of the aforementioned composition would pose the risk that
a) cells of the oral mucosa would also be affected, and
b) non-pathogenic (and possibly even symbiotic) microorganisms would also be attacked.

The term "exposure time usually employed" refers to estimated exposure times with proper use of the preparations according to the invention. As already noted above, said preparations according to the invention are preferably dosage forms such as mouth wash, tinctures, ointments, toothpastes, lozenges, gels and/or rinses, for which the contact time of the active substances or active extracts with the bacteria, or with the mucosal cells, is of the order of a few minutes.

It should be pointed out that with proper use of the aforementioned preparations, the exposure time - i.e. the time during which the body surface to be treated or the established or colonizing microorganisms are exposed to the composition - as a rule does not exceed 10 minutes. A person skilled in the art will be aware that for certain dosage forms the exposure time in proper use could be greater or less than this.

It is therefore important to note that with these exposure times, despite concentrations that would have antibacterial action in continuous exposure, the composition according to the invention develops exclusively an antiadhesive action, without any direct antibacterial and cytotoxic action.

| **Extract** | **Time of action** | **Test cells** | **toxic/bactericidal starting from** | **Fig** |
|---|---|---|---|---|
| MF1, MF2, MF4 | 24 h | KB cells | 100 µg/ml | 5 |
| MF3 | 24 h | KB cells | - | 5 |
| RA 1, RA2, RA4 | 24 h | KB cells | 100 µg/ml | 6 |
| RA3 | 24 h | KB cells | - | 6 |
| MF | 6 h | KB cells | - | 7 |
| RA2, RA4 | 6 h | KB cells | 100 µg/ml | 8 |
| RA1, RA3 | 6 h | KB cells | - | 8 |
| RF | 24 h | KB cells | - | 9 |
| MF1, MF2, MF3 | 24 h | *S. mutans* | 250 µg/ml | 10 |
| MF4 | 24 h | *S. mutans* | 125 µg/ml | 10 |
| RA1, RA2, RA3 | 24 h | *S. mutans* | 250 µg/ml | 11 |
| RA4 | 24 h | *S. mutans* | 125 µg/ml | 11 |
| MF 1, MF2, MF3 | 0.5 h | *P. gingivalis* | - | 12 |
| MF4 | 0.5 h | *P. gingivalis* | 1000 µg/ml | 12 |
| RA1, RA2, RA3 | 0.5 h | *P. gingivalis* | - | 13 |
| RA4 | 0.5 h | *P. gingivalis* | 250 µg/ml | 13 |
| MF1, MF2, MF3 | 24 h | *P. gingivalis* | 125 µg/ml | 14 |
| MF4 | 24 h | *P. gingivalis* | 62.5 µg/ml | 14 |
| RA1, RA2, RA3 | 24 h | *P. gingivalis* | 125 µg/ml | 15 |
| RA4 | 24 h | *P. gingivalis* | 62.5 µg/ml | 15 |

Based on this information, for a person skilled in the art it is a matter of pure routine, for a preparation according to the invention, to adjust the concentration of said composition so that, with a usual exposure time, it does not have antimicrobial and/or bactericidal action. A person skilled in the art is able, in particular, to anticipate for each type of preparation, the expected exposure time in proper use, and adjust the concentration accordingly.

Moreover, it is envisaged according to the invention that the stated constituent from the group of proanthocyanidins is an oligomer and/or a polymer.

Oligomer means, hereinafter, di-, tri- and tetrameric compounds. Polymer means, hereinafter, compounds with four or more monomeric units.

For the reasons stated above, it is especially preferably envisaged that the composition has at least one tetrameric or higher oligomeric proanthocyanidin.

It is also preferably envisaged that the aforementioned oral disease is a disease selected from the group comprising gingivitis, periodontitis, caries, ulcer of the oral mucosa (aphtha), and/or gingivostomatitis, or other periodontal diseases.

Furthermore, it is preferably envisaged that the aforementioned microorganism is at least one bacterium.

Preferably it refers to the species *Porphyromonas gingivalis* (gram-negative, facultatively anaerobic) and *Streptococcus mutans* (gram-positive, facultatively anaerobic).

It is also preferably envisaged that said adhesion of at least one microorganism is provided by a bacterial adhesin. These bacterial adhesins are as a rule proteins that enable the bacterium to adhere to structures or cells of the host. Often these adhesins are arranged on bacterial fimbriae or pili. Important adhesins are listed in the following table.

| **Adhesin** | **Organism** | **Target structure** |
|---|---|---|
| Lys-gingipain | *Porphyromonas gingivalis* | Oral mucosal cells |
| Arg-gingipain | *Porphyromonas gingivalis* | Oral mucosal cells |
| Pilin | Gonococci | Urothelial cells |
| Hemagglutinin | *Porphyromonas gingivalis* | Oral mucosal cells |
| Protein F | *Streptococcus pyogenes* | Fibronectin |
| Pertactin | *Bordetella pertussis* | Bronchial mucosal cells |
| Adhesin P1 | *Streptococcus mutans* | Dental enamel |

According to the invention, an antiadhesive preparation is moreover provided that has at least one proanthocyanidin from a plant *Rumex acetosa.* The preferred embodiments described in connection with the aforementioned use are also applicable to the antiadhesive composition mentioned here.

### Examples

The present invention as characterized in the claims is explained in more detail with the figures and examples presented and discussed below. It should be noted that the figures and examples are only of a descriptive character and are not intended to limit the invention in any way.

### 1. Production of the extracts used

### 1.1 Rumex acetosa L.

The extracts and phases used were kindly supplied by Dr. Jennifer Bicker, Münster University. The extraction and processing of the plant material used were carried out according to BICKER (2008). A scheme is presented in Fig. 1.

### 1.2 Myrothamnus flabellifolia Welw.

The extracts and phases used were kindly supplied by Dr. Jennifer Bicker and Mrs. Claudia Engelhardt, Münster University. Extraction of the leaf material and further processing were based on the scheme in Fig. 1.

### 1.3 Extraction of Rumex acetosa L. and Myrothamnus flabellifolia Welw. (according to BICKER, 2008)

The drug material from *Rumex acetosa* L. and *Myrothamnus flabellifolia* Welw. was extracted, with cooling with ice and exclusion of light, with an acetone-water mixture (7:3 v/v) by vortex extraction using the Ultra-Turrax®. For this, in each case, successively, 100 g of plant material was put in 1 L of solvent and extracted for 5 minutes. After the extract thus obtained had been suction-filtered, the residue was submitted to vortex extraction again with fresh solvent mixture, for exhaustive extraction. All the resultant extracts were combined and the solvent was removed in the rotary evaporator. Then the extracts were cooled overnight to 4°C, so that a proportion of their chlorophyll content was precipitated and could be filtered off with suction. The remaining chlorophyll and other lipophilic constituents were removed by shaking with petroleum ether. For this, 1 L of petroleum ether was added per 1 L of aqueous extract. The shaking operations were repeated until the petroleum ether phase was no longer colored. After that, the solvent was removed from the aqueous phase in the rotary evaporator. The resultant raw extract (designated as RA2, or MF2 hereinafter) was freeze-dried and stored at -20°C until further use. Using this method, a total of 2.5 kg of drug material was processed from *Rumex acetosa* L., and 1 kg from *Myrothamnus flabellifolia* Welw. The total yield of constituents soluble in acetone/water was 258.5 g (= 10.3%) for *Rumex acetosa* L. and 187.7 g (= 18.8%) for *Myrothamnus flabellifolia* Welw.

Further shaking of this raw extract with ethyl acetate led to separation into an aqueous fraction (designated as RA3, or MF3 hereinafter) and an ethyl acetate fraction (designated as RA4, or MF4 hereinafter). Both phases were concentrated by evaporation, freeze-dried and stored at -20°C, protected from the light and air, until further use.

For preparation of the MF1 extract, 10.2 g of shoot tips of *Myrothamnus flabellifolia* Welw. was pulverized using a IKA mill and, after adding 40 mL of 50% ethanol, was stirred for one hour at room temperature. Then the suspension was centrifuged at 2100 x g for 5 minutes, the supernatant was collected, the solvent was removed in a rotary evaporator and it was finally freeze-dried.

The RA1 extract was prepared by adding 5 mL of 50% ethanol to 1 g of leaves of *Rumex acetosa* L., which had previously been pulverized in the IKA mill, and boiling for 30 minutes under reflux. The suspension was left to stand overnight at room temperature and then centrifuged for 10 minutes at 3166 x g. To separate residual plant material, the collected supernatant was then centrifuged again at 25 000 x g for 10 minutes. The solvent was removed from the resultant supernatant in the rotary evaporator and finally lyophilization of the extract was carried out.

See Fig. 1 for an overview of the extraction method.

### 1.4 Rhododendron ferrugineum L.

The extracts from *Rhododendron ferrugineum* L. used for the tests were kindly supplied by Mrs. Andrea Louis, Münster University.
To prepare an aqueous extract (RF H₂O), 30 mL of demineralized water was added to 3 g of leaf material of *Rhododendron ferrugineum* L. and it was treated for 5 minutes in the ultrasonic bath. To separate the plant material, the preparation was centrifuged for 5 minutes at 3600 x g. 10 mL of the clear supernatant was then withdrawn and freeze-dried.

In addition, 2 x 1.65 L of demineralized water was added to 250 g leaf material of *Rhododendron ferrugineum* L. and extracted in each case for 20 hours with stirring in the cold room. To separate the plant material, in each case it was centrifuged at 14 800 x g for 10 minutes and the respective supernatants were combined. The solution was concentrated to 600 mL in the rotary evaporator with a water bath temperature below 40°C. The 600 mL obtained was slowly added dropwise to 2.4 L of 96% ethanol and the solution was stored overnight in the cold room for precipitation of the polysaccharides. Then the solution was centrifuged again at 14 800 x g for 10 minutes, the supernatant was removed and the residue was suspended in demineralized water and lyophilized. 2.5 g of the extract obtained was dialyzed using a cellulose membrane (MW CO 3500 Da) and as a result the raw polysaccharide RF RPS B2 was obtained after subsequent freeze-drying.

### 2 Hemagglutination assay

The hemagglutination assay with *P*. *gingivalis* was carried out under sterile conditions in 96-well plates with U-shaped bottoms. It was carried out using a 3-5 days old culture of *P. gingivalis* ATCC 33277, and freshly obtained, human EDTA-whole blood. For preparation of a 2% erythrocyte suspension, the blood was transferred to sterile 15 mL Falcon tubes and centrifuged at 4°C for 10 minutes at 1200 x g. The clear, colorless plasma supernatant was then removed and the erythrocytes that remained were washed with PBS solution. Three to five washing steps were carried out, until the supernatant was colorless and a definite sediment of erythrocytes had formed. For counting the erythrocytes, an aliquot was contain from the sediment and diluted 1:100 with PBS solution. 20 µL of this dilution was applied on a Neubauer counting cell and 16 large squares were counted by microscope at 100 x magnification. Based on the erythrocyte count obtained, a 2% suspension in PBS solution was prepared.
The freshly grown culture of *P. gingivalis* was resuspended in an amount of PBS solution corresponding to the sample size, and was adjusted to OD₆₆₀ = 2.0 against pure PBS.

For each microtiter plate, an erythrocyte control series (negative control, consisting of erythrocyte suspension and PBS solution), an untreated control (positive control, consisting of erythrocyte suspension and *P. gingivalis* suspension), and a sample control for testing for autoagglutination by the test substance (sample control, consisting of erythrocyte suspension and test solution) were carried out together.

Using a multiple-channel pipet, aliquots of 25 µL of sterile PBS solution was prepared and 25 µL of the *P. gingivalis* suspension was added. Serial dilution was carried out (1:2, 1:4, 1:8, etc.) with 25 µL and then 25 µL of the test solution was added, or in the case of the positive control, 25 µL of PBS solution was added. Determination was carried out in duplicate for each test substance. The preparations were mixed for 5 minutes on a horizontal shaker and then incubated at 37°C for 30 minutes.

After this incubation, 50 µL of the 2% erythrocyte suspension was added to each well, so that the final volume per well corresponded to 100 µL. The preparations were mixed once again for 5 minutes on the horizontal shaker and incubated for 2 to 3 hours at room temperature, until a definite erythrocyte sediment could be seen in the negative control. Provided this control, as well as the sample control did not display any hemagglutination, the test could be evaluated. Hemagglutination appeared as a diffuse, reddish solution in the well, whereas absence of agglutination and thus sedimentation of the erythrocytes could be seen as a clearly defined, red spot in the surrounding, clear PBS solution, also called "button formation".

For the evaluation, the titers were read off, as the reciprocal of the dilution that still displayed hemagglutination, and were assessed in comparison with the untreated control. This evaluation was carried out both visually and by means of the MicroWin® software.

### 2.1 Incubation variant of the hemagglutination assay

This incubation variant was in particular carried out when test substances caused autoagglutination of the erythrocytes.

In this incubation variant, *P. gingivalis* cells were first preincubated with the test solution. For this, 150 µL of the *P. gingivalis* suspension OD₆₆₀ = 2.0 was mixed with 150 µL of the test solution, or with 150 µL of PBS solution in the case of the positive control in a sterile 1-mL reaction vessel and incubated for 30 minutes at 37°C. After this preincubation, the preparations were centrifuged for 5 minutes at 6000 x g, the supernatant was discarded and the cell pellet obtained was resuspended in 300 µL PBS solution. Using a multiple-channel pipet, 25 µL aliquots were put in the wells of a 96-well microtiter plate and 25 µL of the resuspended *P. gingivalis* suspension was added. As in the original incubation method, serial dilution of the bacterial suspension was carried out. Finally 25 µL of PBS solution and 50 µL of the 2% erythrocyte suspension were added per well and the preparation was shaken gently for 5 minutes horizontally at 160 rpm. The evaluation was also carried out as in the original test method.

### 2.2 Results of the hemagglutination assay

Table 1 shows the results of the hemagglutination assay (n = 2). This shows the inhibition of erythrocyte agglutination in titer stages (logarithmic dilution stages) .

**Table 1**

| **Test substance** | **Concentration [µg/mL]** | | | |
|---|---|---|---|---|
| | 1000 | 100 | 50 | 1 |
| RF2 | - | 2 | 1 | 0 |
| RF RPS B2 | 4 | 3 | - | 0 |

Table 2 shows the results of the hemagglutination assay with preincubation of *P. gingivalis* with the test substances (n=2-4). This shows the inhibition of erythrocyte agglutination in titer stages.

**Table 2**

| **Test substance** | **Concentration [µg/mL]** | | | |
|---|---|---|---|---|
| | 500 | 100 | 50 | 1 |
| MF1 | 1.7 | 1.6 | 1.4 | 0.5 |
| RA2 | 2 | - | - | 0 |
| RA3 | 2 | 1 | - | 0 |
| RA4 | 1 | 2 | - | 0 |

### 2.3. Fluorescence activated cell sorting (FACS) assay

Bacterial cells from a 3-5 days old culture of *P. gingivalis* (ATCC 33277) were first resuspended in 0.5 M NaHCO₃ buffer (pH 8.0) and the optical density of the cell solution was adjusted to OD₆₆₀ = 4.0. FITC-labeling was carried out with a concentration of 50 µg/mL over a period of 30 minutes at 37°C. Then excess FITC was removed by washing twice (3600 x g, 5 minutes) with PBS. This was followed by various incubation variants (see 3.1). After incubation, nonadhering bacteria were removed by washing three times with PBS and the cells were detached from the plate surface by adding trypsin/ EDTA (0.05% + 0.02% EDTA in PBS) and washed again with PBS. The cell pellet was resuspended in 1 mL PBS and analysis of adhesion was carried out by FACS. The strength of adhesion of P. gingivalis to the KB cells was determined by means of the CellQuest software (from BectonDickinson).

### 2.4 Incubation variants

All incubation variants were carried out on KB cells in 6-well plates, which were approx. 80-100% confluent.

### 2.4.1 Preincubation of P. gingivalis with test substance

FITC-labeled cells of *P. gingivalis* (OD₆₆₀ = 1.0) were incubated with the test substances for 30 minutes at 37°C. The substances were then removed by single washing of the bacterial cells with PBS (3600 x g, 5 minutes).
Shortly before adding the *P. gingivalis* cells (BCR 100:1), previously sown KB cells (ATCC CCL-17) were washed once with PBS and then incubated with FCS-free KB cell medium. The KB cells were incubated with the bacteria for 90 minutes at 37°C.

### 2.4.2 Preincubation of the KB cells with test substance

The medium of previously sown KB cells (ATCC CCL-17) was removed and the cells were washed once with PBS. The KB cells were incubated with test substance in FCS-free cell medium for a period of 1.5 hours. Then the KB cells were washed with PBS and FITC-labeled *P. gingivalis* was added in a BCR of 100:1. Incubation with the bacteria was carried out for 90 minutes at 37°C.

### 2.4.3 Coincubation of KB cells with P. gingivalis and test substance

KB cells (ATCC CCL-17) were incubated in FCS-free medium with test substance and FITC-labeled cells of *P. gingivalis* (BCR 100:1) for a period of 90 minutes.

### 2.4.4 Results of the FACS-assay

### 2.4.5 Results of the FACS-assay with preincubation of P. gingivalis with the test substances

Table 3 shows the results of the FACS-assay with preincubation of *P. gingivalis* with the test substances. It shows the adhesion of *P. gingivalis* to the KB cells in [%]. Untreated control = 100%. (n=2-5)

**Table 3**

| **Test substance** | **Concentration [mg/mL]** | | | |
|---|---|---|---|---|
| | 1 | 0.5 | 0.1 | 0.01 |
| MF1 | 49 ± 14 | 62 ± 15 | 89 ± 16 | |
| MF2 | 38 ± 27 | 48 ± 18 | 82 ± 10 | 100 ± 5 |
| MF3 | 50 ± 22 | 61 ± 14 | 80 ± 22 | 91 ± 24 |
| MF4 | 44 ± 29 | 43 ± 8 | 73 ± 14 | 91 ± 21 |
| RA1 | 76 ± 13 | - | - | - |
| RA2 | 75 ± 10 | 80 ± 19 | 80 ± 38 | 78 ± 10 |
| RA3 | 78 ± 14 | 91 ± 16 | 101 ± 6 | 93 ± 10 |
| RA4 | 71 ± 27 | - | - | - |

| **Test substance** | **Concentration [mg/mL]** | | | |
|---|---|---|---|---|
| | 10 | 5 | 1 | 0.1 |
| RF H₂O | 70 ± 14 | 68 ± 13 | 78 ± 12 | 97 ± 4 |
| RF RPS B2 | - | - | 61 ± 19 | 100 ± 5 |

### 2.4.6 Results of the FACS-assay with preincubation of the KB cells and test substance

Table 4 shows the results of the FACS-assay with preincubation of the KB cells with the test substances. It shows the adhesion of *P. gingivalis* to the KB cells in [%]. Untreated control = 100% (n=3-5). See also Fig. 2.

**Table 4**

| **Test substance** | **Concentration** |
|---|---|
| | **0.1 mg/mL** |
| MF1 | 77 ± 21 |
| RA2 | 81 ± 21 |
| RF RPS B2 | 100 ± 3 |
| RF H₂O | 91 ± 7 |

### 2.4.7 Results of the FACS-assay with coincubation of the KB cells with P. gingivalis and test substance

Table 5 shows the results of the FACS-assay with coincubation of the KB cells with the test substances and *P. gingivalis.* It shows the adhesion of *P. gingivalis* to the KB cells in [%]. Untreated control = 100% (n=3-5). See also Fig. 3.

**Table 5**

| **Test substance** | **Concentration [mg/mL]** | | |
|---|---|---|---|
| | 0.1 | 0.05 | 0.1 |
| MF1 | 85 ± 3 | 85 ± 10 | 93 ± 10 |
| RA2 | 84 ± 7 | - | - |
| RF RPS B2 | 96 ± 3 | - | - |
| RF H₂O | 91 ± 3 | - | - |

### 3. Influence of the test substances on biofilm formation of Streptococcus mutans

To test the influence of the test substances on biofilm formation of *S. mutans,* a biofilm model was developed. For this, a 24-hour culture of *S. mutans* ATCC 25175 was prepared in BHI medium with 1.5% sucrose. From this preculture, after 24 hours, 10 mL of the BHI medium with a cell count of 7.5 x 10³ CFU was inoculated in a 50 mL Falcon tube, which contained 10 µg/mL of the test substance, or in the case of the untreated control (negative control), no test substance was added. Sterile glass rods with a diameter of 5 mm, whose weight was determined beforehand and on which biofilm formation takes place, were immersed vertically in the culture to a depth of 1 cm. Incubation was carried out for 72 hours at 37°C, changing the medium (negative control), or changing the medium with test substance, every 24 hours. After incubation for three days, the glass rods were dried for an hour at room temperature and their weight was determined. The biomass that had formed was found by subtracting the blank weight from the final weight. See also Fig. 4.

### 4. Influence of the test substances on the vitality of KB cells

To investigate the influence of the test substances used on vitality, the KB cells were first sown in 96-well tissue culture plates (F-shape) with 1.5 x 10⁴ cells/well and incubated for 24 to 48 hours at 37°C. In this time, the cells adhere to the plate surface. At approx. 70% confluence, the medium was removed and the cells were washed once with PBS solution (200 µL/well). Corresponding dilutions of the test substances in FCS-free EMEM were prepared in small Petri dishes, which were then put in the individual wells of the microtiter plate. A 12-fold determination was carried out for each substance per test series. The KB cells were incubated with the test substances for 6 and 24 hours at 37°C. As a negative control, cells were incubated with pure, FCS-free EMEM.

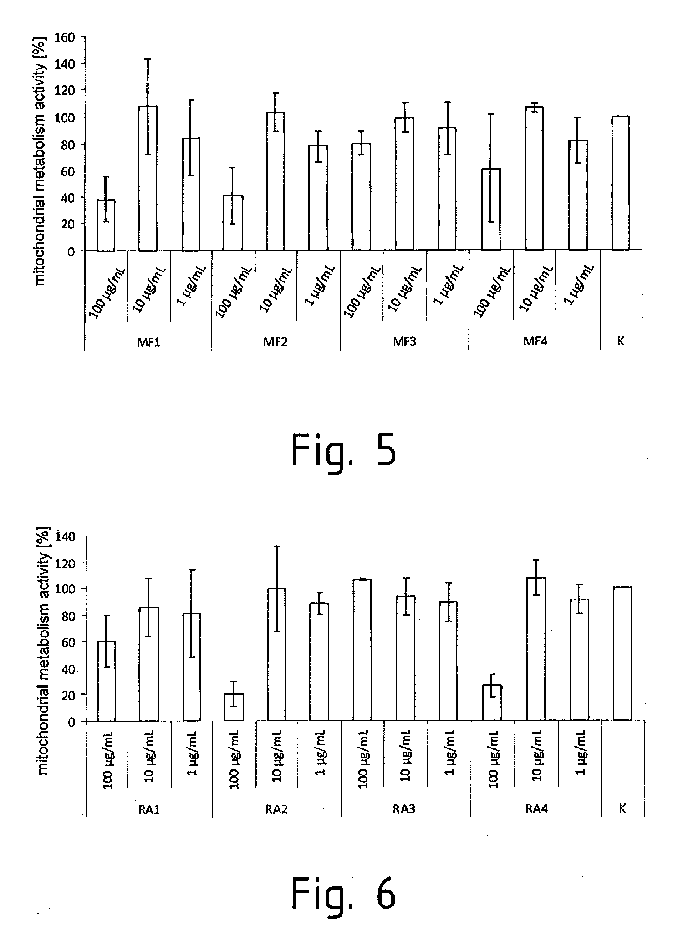

After incubation, an MTT test was carried out for determining the mitochondrial dehydrogenase activity. For this, first the supernatant medium was removed from the individual wells of the microtiter plate and the cells were washed once with PBS (200 µL/well). Then 50 µL MTT-reagent (2.5 mg/mL PBS) was added per well and the plates were incubated at 37°C for 4 hours. After this incubation, the MTT-reagent was removed by striking the plate and 50 µL DMSO per well was added to dissolve the formazan crystals that had formed. Photometric evaluation of the test was carried out after approx. 10-minute shaking in the TECAN-reader at λ = 492 nm against the reference wavelength λ = 690 nm. See also Figs. 5 - 9.

### 5. Influence of the test substances on the vitality of P. gingivalis and S. mutans

The influence of the test substances on the vitality of *P. gingivalis* and *S. mutans* was determined by MTT-tests. For testing for *P. gingivalis,* starting from a three days old culture, a liquid culture was prepared with OD₆₆₀ = 0.05. In the exponential growth phase, i.e. after approx. 24 hours of incubation at 37°C, this culture was used for subsequent tests, for which it was adjusted to OD₆₆₀ = 0.25 in liquid medium.

For testing for *S. mutans,* a 24-hour culture was also prepared (BHI medium with 1.5% sucrose). This was then adjusted for subsequent tests to OD₆₀₀ = 0.001 in BHI medium.

For carrying out the MTT-tests, sterile, 96-well microtiter plates, F-shape, were used. First, 25 µL aliquots were put in the individual wells of the microtiter plate and 25 µL of the test substance dissolved in the respective culture medium was added. The substance solution was then diluted serially in each case to 25 µL with a multiple-channel pipet (1:2, 1:4, 1:8, etc.) and the last 25 µL was discarded. Then 25 µL of the respective culture medium was added to the wells and 50 µL of the corresponding suspension of *P*. *gingivalis,* or *S. mutans.* In the case of the substance control, culture medium was added instead of the bacterial suspension. In each test series, a negative control was also conducted in parallel, without using any test substance, but only growth of the bacteria in the culture medium. The total volume of each well in the individual wells was therefore 100 µL.

For carrying out the MTT-tests with *P. gingivalis,* 40 µL of MTT-reagent (2.5 mg/mL PBS) was put in the individual wells of the microtiter plate and the preparations were incubated for 30 minutes at 37°C. Photometric measurement was carried out in the TECAN-reader at λ = 490 nm.
For carrying out the MTT-tests with *S*. *mutans,* the supernatant in the 96-well microtiter plates was removed with a multiple-channel pipet and 50 µL of M T T-reagent (2.5 mg/mL PBS) was put in the individual wells. The preparations were incubated for 30 minutes at 37°C and the MTT-reagent was then poured off. The formazan crystals that formed were dissolved by adding 50 µL DMSO and photometric measurement was carried out at λ = 490 nm against the reference wavelength of λ = 630 nm after shaking the plates for 5 minutes.

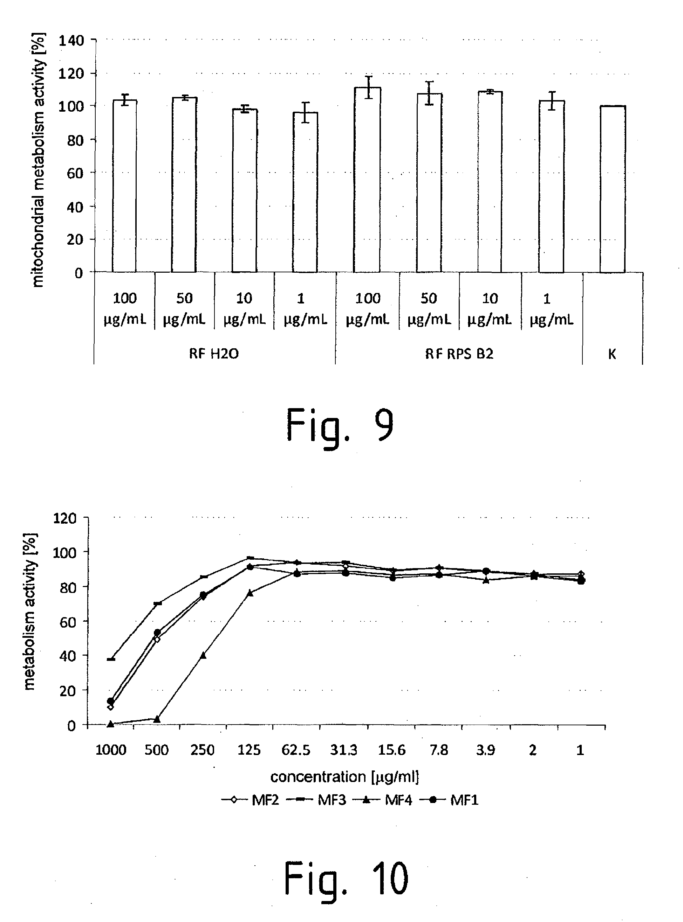

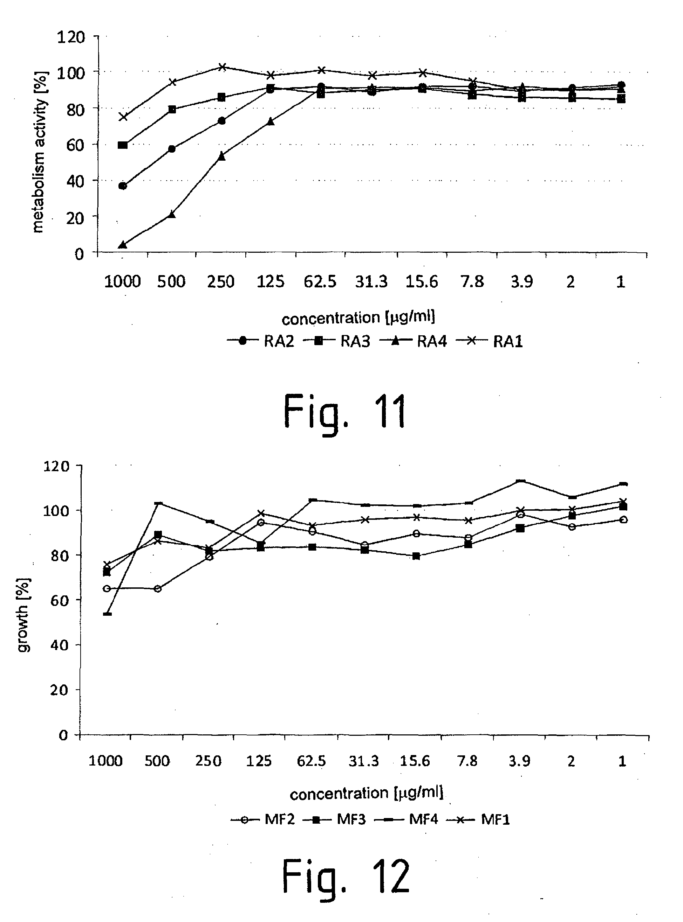

Figs. 10 - 11 show the results for toxicity of the test substances on S. mutans after 24 hours incubation. Figs. 12 - 15 show the results for toxicity of the test substances on P. *gingivalis* after 30 minutes and 24 hours incubation.

### 6. Effect of the test substances on gene expression on various inflammation markers

### 6.1. Incubation method

The KB cells were first sown in FCS-containing EMEM in 6-well plates at a concentration of 1.5 x 10⁵ cells/well and were incubated for approx. 3 to 5 days to confluence of approx. 80% at 37°C.

A liquid culture of *P. gingivalis* ATCC33277 with OD₆₆₀ = 0.05 was prepared one day before carrying out the experiment.

Before adding the test substance to the KB cells, the cell medium was removed and the wells of the plates were washed once with PBS solution. Then, in each well, 2 mL of FCS-free EMEM containing the dissolved test substance was added to the KB cells and incubated for two hours at 37°C. The untreated control was left in pure, FCS-free EMEM.

Before incubation with *P. gingivalis,* first the cell culture medium in the 6-well plates was removed and the KB cells were washed once with PBS solution. From the overnight culture of *P. gingivalis,* a cell suspension was prepared in FCS-free EMEM, which corresponded to a BCR (bacterial cell ratio) of 5:1 to the KB cells in the 6-well plates and was put in the individual wells. The incubation time was two hours.

### 6.2. Isolation of mRNA

After removing the cell culture medium with *P. gingivalis* and washing three times with PBS, the cells were detached from the bottom of the well by adding 750 µL trypsin (0.05% + 0.02% EDTA in PBS). After transfer to sterile 2 mL reaction vessels and 5-minute centrifugation at 100 x g, the cells were once again washed with 1 mL PBS and the cell pellet obtained after further centrifugation was quick-frozen in liquid nitrogen and stored at -80°C until further processing. The mRNA was isolated from the cell pellet using the NucleoSpin® RNA XS Kit from the company MACHERY-NAGEL, Düren, Germany.

First, for cell disruption, 100 µL RA1 buffer, and 2 µL TCEP (tris(2-carboxyethyl)phosphine) were added to the cell pellet and vortexed twice for 5 seconds. After adding 5 µL of Carrier RNA Working Solution (20 ng) and vortexing again, the suspension could be transferred to the NucleoSpin® filter and was centrifuged with this for 30 seconds at 11 000 x g. 100 µL of 70% ethanol was added to the filtrate, mixed carefully and applied to the NucleoSpin® RNA XS column. After centrifuging again for 30 seconds at 11 000 x g, the membrane was dried by adding 100 µL MDB (membrane desalting buffer) and centrifugation for 30 seconds at 11 000 x g. This was followed by 15-minute DNAse digestion at room temperature by adding 3 µL rDNase and 27 µL reaction buffer for rDNase to the membrane. Next there were three washing steps, first adding 100 µL RA2 buffer to the membrane for 2 minutes to inactivate the rDNase. After centrifugation for 30 seconds at 11 000 x g, 400 µL RA3 buffer was applied to the membrane and centrifuged under the same conditions. The last washing step was carried out by adding 200 µL RA3 buffer and two-minute centrifugation at 11 000 x g. The NucleoSpin® RNA XS column was then put in a 1.5 mL reaction vessel and the mRNA bound to the membrane was detached by adding 25 µL of RNase free water. Elution was effected by centrifugation at 11 000 x g for 30 seconds.

### 6.3. Quantification of the RNA content

The RNA obtained after isolation was diluted 1:10 with RNase free water and measured at λ = 260 nm and λ = 280 nm on the biophotometer. An OD₂₆₀ of 1.0 corresponds to a concentration of 40 µg/mL RNA. The quotient of the measurement at λ = 260 nm and λ = 280 nm (OD₂₆₀/OD₂₈₀) serves as a measure of the purity and should be between 1.8 and 2.0. Lower values indicate contamination with proteins.

### 6.4. Reverse transcription

The high-capacity cDNA reverse transcription kit from the company Applied Biosystems, Foster City, USA, was used.

For each preparation, a mixture of 2 µL 10 x rT-PCR buffer, 0.8 µL 25x dNTP mix, 2 µL random primer, 1 µL MultiScribe transcriptase and 1 µL RNase inhibitor was put in RNase free, sterile 0.2 mL reaction vessels, 1 µg of the target RNA was added and was made up with RNase free water to a total volume of 20 µL.
Reverse transcription was carried out according to the temperature program given in Table 6 in the Mastercycler from the company Eppendorf, Hamburg, Germany.

Table 6 shows the temperature program for the reverse transcription (High Capacity Kit, Applied Biosystems)

**Table 6**

| | |
|---|---|
| 25°C | 10 minutes |
| 60°C | 37 minutes |
| 85°C | 5 seconds |

### 6.5. Real-time polymerase chain reaction (Real Time PCR)

For each preparation, 1 µL of the corresponding TaqMan gene expression assay (20x) with 10 µL TaqMan Universal MasterMix (2x, without Amperase) and 9 µL of the target cDNA (100 ng) were put in a 96-well optical reaction plate and centrifuged briefly. To avoid evaporation effects, the plate was sealed with an optical film and Real Time PCR was carried out using the 7300 Real Time PCR System from the company Applied Biosystems, Foster City, USA according to the temperature program shown in Table 7. Evaluation was carried out with the 7300 System Sequence Detection Software, version 1.2.3.

Table 7 shows the temperature program for real time PCR

**Table 7**

| | | | |
|---|---|---|---|
| Enzyme activation | 96°C | 10 minutes | |
| Denaturation | 95°C | 15 seconds | 45 cycles |
| Annealing/extension | 60°C | 60 seconds | |

### 6.6. Primers for investigating gene expression

The primers for the gene expression analyses were obtained from Applied Biosystems, Foster City, USA and are shown in Table 8.

Table 8 shows the assay-IDs of the primers used for gene expression analysis in KB cells, obtained from the company Applied Biosystems, Foster City, USA.

**Table 8**

| | |
|---|---|
| IL-1β | Hs01555413_m1 |
| IL-6 | Hs99999032_m1 |
| IL-8 | Hs01567913_g1 |
| TNFα | Hs00174128_m1 |
| 18s RNA | Hs99999901_s1 |

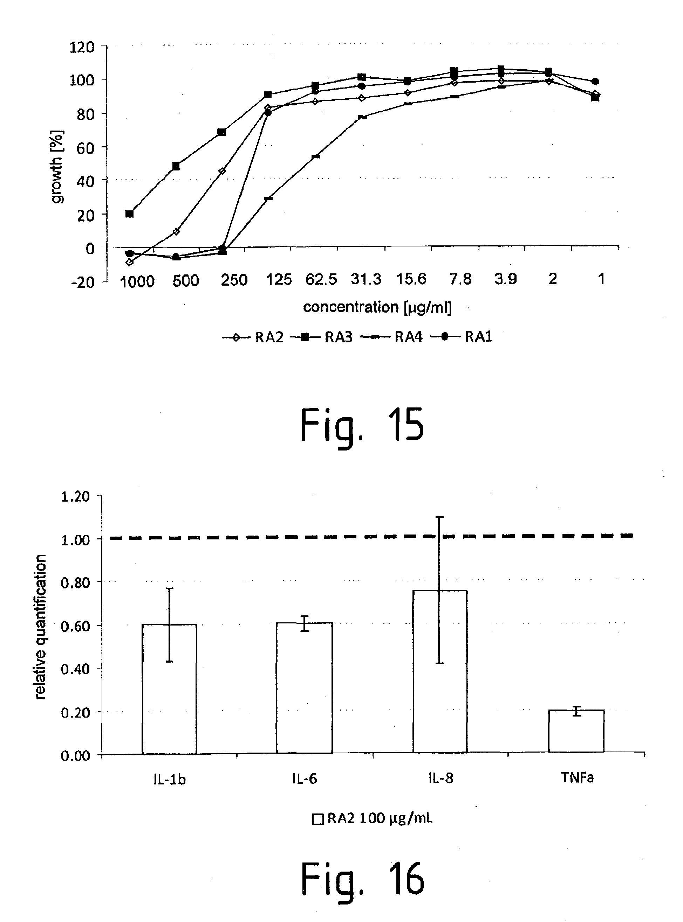

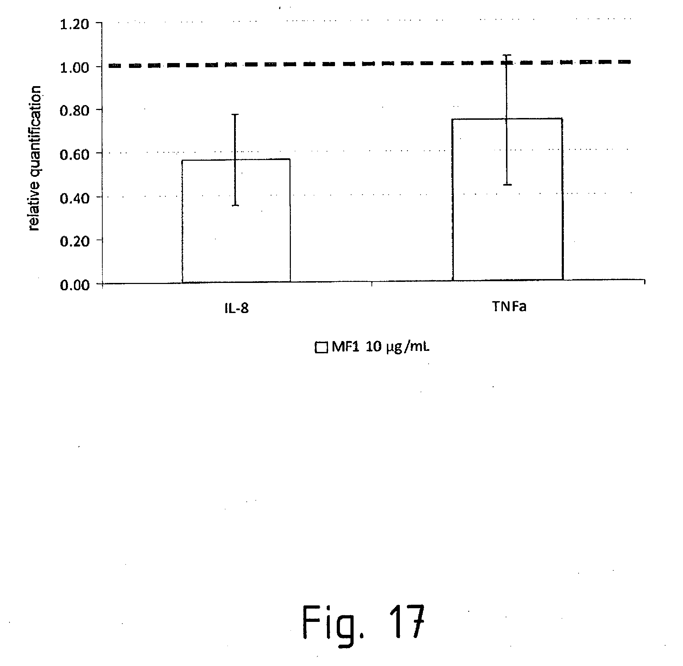

Figs. 16 and 17 show the results of the Real Time PCR analyses

### 6.7. Appendix

Adherent KB cells were incubated at 37°C and 5% CO₂ in 25- or 75-cell culture bottles. Cultivation of *P. gingivalis* took place under anaerobic conditions (10% H₂, 10% CO₂, 80% N₂) at 37°C on special blood agar plates or in liquid culture. The anaerobic atmosphere was achieved by adding anaerobic bags (AnaeroGen™) in an anaerobic jar, and was verified by adding a redox indicator strip (Anaerotest®).

### 6.8. Recipes

### KB medium

| | |
|---|---|
| MEM Eagle-EBSS with NEAA w/o CaCl₂ | 450 mL |
| Gentamicin | 50 µg/mL |
| FCS | 8% |

### Porphyromonas gingivalis-agar

| | |
|---|---|
| Agar-agar | 16 g |
| Trypticase peptone | 15 g |
| Neutralized soya peptone | 5 g |
| NaCl | 5 g |
| Yeast extract | 5 g |
| Cysteine | 0.5 g |
| Vitamin-K₁ stock solution | 1 mL |
| Hemin stock solution g | 10 mL |
| Sheep's blood | 50 mL |

| | |
|---|---|
| to 1 L with doubly-distilled water | |

### Porphyromonas gingivalis-liquid medium

| | |
|---|---|
| Trypticase peptone | 15 g |
| Neutralized soya peptone | 5 g |
| NaCl | 5 g |
| Yeast extract | 5 g |
| Cysteine | 0.5 g |
| Vitamin-K₁ stock solution | 1 mL |
| Hemin stock solution | 10 mL |

| | |
|---|---|
| to 1 L with doubly-distilled water, autoclave | |

### Streptococcus mutans-agar

| | |
|---|---|
| BHI | 37 g |
| Sucrose | 0.5% |
| Agar-agar | 15 g |

| | |
|---|---|
| to 1 L with doubly-distilled water, autoclave | |

### Streptococcus mutans-liquid medium

| | |
|---|---|
| BY THE FOLLOWING | 37 g |
| Sucrose | 1.5% |

| | |
|---|---|
| to 1 L with doubly-distilled water, autoclave | |

### Vitamin-K₁ stock solution

| | |
|---|---|
| Vitamin-K₁ | 100 µL |
| Ethanol (96%) | 9.9 mL |

| | |
|---|---|
| store at 4°C in the dark | |

### 7. Additional Experiments

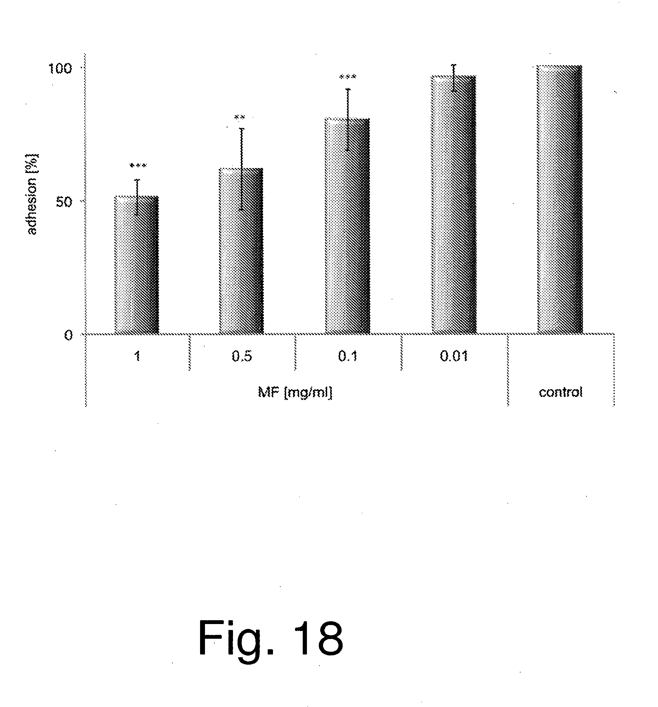

Additional experiments used for illustrative purpose of the present invention only, were carried out with a slightly different extract from *M. flabellifolia* termed just "MF" in the following descriptions and in the Figures 18-23. This extract MF is very similar to the above described MF1, however, this time the extractionw as carried out according to Anke et al. 2008

### 7.1 Materials and Methods

### 7.1.1.Extract from M. flabellifolia MF

Plant material (Myro AG, Switzerland, batch Myrol) (Anke et al, 2008) was powdered. 10 g were extracted under stirring with EtOH:water (1:1 v/v) for 30 min at RT and the suspension centrifuged (10.000 × g, 10 min). The supernatant was lyophilized to yield the extract named MF. Standardisation of the extract and analytical quality control was done by ultrahigh pressure liquid chromatography (UPLC) by quantitation of the lead compounds of the extract (trigalloyl quinic acid, tetragalloyl quinic acid, tellimagrandin II) and of the proanthocyanidin content (Anke et al, 2008).

### 7.1.2 P. gingivalis strain and growth conditions

*P. gingivalis* (ATCC 33277) was cultured under anaerobic conditions (Anaerocult, Merck, Darmstadt, Germany) at 37°C on solid sheep blood agar (per 1 L: 16 g agar, 15 g trypticase peptone, 5 g neutralised soja peptone, 5 g sodium chloride, 5 g yeast extract, 0.5 g L-cysteine, 50 ml sheep blood) enriched with 10 mg vitamin K and 5 mg hemin. For quality control purposes (identity and purity), every third passage of the bacterium was subjected to quantitative *fimA* gene PCR analysis.
Bacteria used in assays were grown to late log phase, harvested and used to inoculate 20 ml of liquid media (the same as for solid medium without blood and agar) and grown overnight at 37°C. Growth was monitored by OD (660 nm) and bacteria in exponential phase were used for further experiments.

### 7.1.3 Cell culture

KB cells (ATCC CCL-17), derived from a human oral epidermoid carcinoma, were kindly provided by Dr. S. Eick (University of Jena, Germany). The cells were cultured in Earl's minimum essential medium (EMEM) (Lonza, Basel, Switzerland), supplemented with 8% (v/v) heat-inactivated FCS (PAA Laboratories, Cölbe, Germany) and 50 µg/ml gentamicin (MP Biomedicals, Irvine, USA) at 5% CO₂ / 37°C. Passaging was performed twice a week to a maximum of 15 passages.

### 7.1.4 Adhesion assay with P. gingivalis and KB cells

KB-cells (8×10⁵ cells) were grown to 90 % confluence in 6-well plates. The supernatant was removed, the monolayer washed with PBS and incubated in FCS- and antibiotic-free medium. Solid medium-grown *P. gingivalis* bacteria (3 to 5 days culture) were harvested and suspended in 0.5 M NaHCO₃, pH 8.0. After adjusting the OD₆₆₀ to 4.0, fluorescein isothiocyanate FITC (50 µg/ml) (Sigma-Aldrich, Steinheim, Germany) was added for labelling of the bacteria followed by incubation for 30 min / 37°C. Bacteria were pelleted (3.600 × g, 5 min) and washed twice in PBS to remove unbound FITC. FITC-labelled bacteria were suspended in EMEM. Labelling efficiency was analyzed by fluorescence microscopy. To investigate the effect of MF on the adhesion of *P. gingivalis* on KB cells, various incubation procedures were applied.
i. For *preincubation of labelled bacteria,* bacteria were mixed with MF (1, 0.5, 0.1 and 0.01 mg/ml) and incubated for 30 min at 37°C. After pelleting and washing with PBS, bacteria were resuspended in EMEM and added to KB cells (BCR 100:1) for 90 min at 37°C.
ii. For *preincubation of KB cells,* MF (0.1 mg/ml) was added to EMEM and the cells were incubated for 90 min at 37°C. Subsequently, KB cells were washed vigorously three times with PBS and labelled *P. gingivalis* were added (BCR 100:1) and incubated for 90 min at 37°C.
iii. For *coincubation,* MF (0.1 mg/ml) and FITC-labelled *P. gingivalis* (BCR 100:1) were simultaneously added to KB cells and incubated for 90 min at 37°C.
After each incubation variant, KB cells were washed three times with PBS, detached from the wells by incubation with trypsin-EDTA for 3 min at 37°C and then suspended in EMEM, supplemented with FCS (8%). The adhesion intensity of FITC-labelled *P. gingivalis* on KB cells was immediatly analyzed by flow cytometry (FACS Calibur, Becton Dickinson, Heidelberg, Germany). Instrument settings were as follows: FCS (Detector): E-1 (Voltage), 3.07 (Amp Gain), Lin (Mode); SSC: 332, 1.00, Lin; FL1: 360, 1.00, Log.

### 7.1.5 Gene expression analysis in KB cells

KB-cells (8×10⁵ cells) were grown to 90 % confluence in 6-well plates. Prior to incubation with *P. gingivalis,* the supernatant was removed, monolayers were washed with PBS and incubated in FCS- and antibiotic free media (EMEM). MF (10, 100 µg/ml) was added and cells were incubated for 2 h / 37°C. Cells were isolated, washed three times with PBS and further incubated in EMEM.
24 hours prior to the experiment, a bacterial liquid culture was inoculated with solid medium-grown *P. gingivalis* (OD₆₆₀ = 0.05) and incubated into exponential phase. Bacteria were pelleted (3.600 × g, 5 min) and resuspended in EMEM. The OD₆₆₀ of the bacterial suspension was adjusted to a BCR of 5:1 and added to the KB cells for an incubation period of 2 h at 37°C. Following that, the supernatants were removed, KB cells were washed three times with PBS and subsequent trypsinized (see above). After collecting the KB cells by centrifugation (100 × g, 5 min), mRNA was isolated using the NucleoSpin® RNA XS Kit (Machery-Nagel, Düren, Germany) according to manufacturer's instructions.
The total RNA concentration in each sample was calculated from the OD₂₆₀. cDNA was synthesized from 1 µg of total RNA by using the High-Capacity cDNA Reverse Transcription Kit (ABI, Germany) according to the manufacturers' instructions.
RT PCR analysis was performed by 7300 RT PCR (ABI, Germany). 2 µl (100 ng) cDNA were added to 1 µl 20 × gene expression assay (ABI, Germany) (assay ID's see Table 2), 10 µl TaqMan Universal MasterMix (2 ×, w/o amperase) (ABI, Germany) and 7 µl RNase free water in an optical 96-well plate. Enzyme activation for 10 min at 96°C was followed by 45 cycles of denaturation for 15 sec at 95°C and annealing/extension for 60 sec at 60°C. mRNA levels were expressed as RQ-values, related to the C_{T}-value of the 18S rRNA and to the control (KB cells, not pretreated with MF, only infected with *P. gingivalis*).

### 7.1.6 Gene expression analysis of P. gingivalis (RT PCR)

Solid medium-grown *P. gingivalis* from a 3 days culture were harvested and used to inoculate liquid media (OD₆₆₀ = 0.05). After 24 h bacteria were transferred to 4 ml liquid culture media (OD₆₆₀ = 0.1). MF was added at different concentrations (10, 100 µg/ml). Liquid culture with *P. gingivalis* served as negative control. After incubation for 6 h at 37°C bacteria were harvested by centrifugation (11.000 × g, 5 min) and washed with PBS. The pellet was resuspended in PBS (100 µl) and mixed with TE-Buffer (500 µl), 1% SDS, phenol and lysing matrix B (0,4 g) (MP Biomedicals, Irvine, USA). Cells were lysed by FastPrep®-24-System (MP Biomedicals, Irvine, USA) (2 × 50 sec, level 5.0) and RNA-isolation was followed by phenol-chloroform-extraction and ethanol-precipitation. Removal of DNA was performed by NucleoSpin®rDNase (Machery-Nagel, Düren, Germany) according to the manufacturer's instructions. Total RNA concentration in each sample was calculated from OD₂₆₀. cDNA was synthesized from 1 µg of total RNA by using the QuantiTectReverse Transcription System (Qiagen, Hilden, Germany). RT-PCR was performed with 2 µl (100 ng) cDNA, 10 µl 2 × QuantiTect Probe PCR Master Mix (Qiagen, Hilden, Germany), 1 µl 20 × Primer Mix, 1 µl 20 × QuantiProbe Solution (sequences listed in Table 1) and 6 µl RNase free water. The oligonucleotide primers and probes (Table 1) were synthesized by Qiagen (Düsseldorf, Germany).

PCR was performed on a 7300 Real Time PCR System (Applied Biosystems (ABI), Germany) and 7300 System Sequence Detection Software (Version 1.2.3, ABI, Germany). A total of 45 cycles were performed after 15 min at 95°C for enzyme activation. Elongation at 76°C / 30 sec, denaturation at 94°C / 15 sec, annealing at 56°C / 30 sec. mRNA levels were expressed as RQ-values, related to the C_{T}-value of the 16S rRNA and to the untreated control.

### 7.1.7 Genome-wide gene expression analysis by microarray hybridization

The microarrays were designed and synthetisized by NimbleGen^{®}. Each open reading frame of the complete *P. gingivalis* W83 genome was represented on the array. If applicable, each open reading frame was represented by thirteen 60-mer oligonucleotides, and each oligonucleotide was present in three replicates randomly located on the microarray to ensure utmost reproducibility of the hybridization process and read-out of the fluorescence signals. As negative controls served 3510 random probes spotted at various positions on the chips.

### cDNA synthesis, labelling and hybridization

For gene-array analysis an 4 ml aliquot from a 3 days liquid culture was inoculated into fresh medium to render an OD₆₆₀ 0.05 and cultivated for 12 h. Using this culture, 4 ml with an OD₆₆₀ value of 0.1 were incubated with MF or medium alone (control group) for 6 h. Bacteria were pelleted and washed. Total RNA was isolated and DNA was removed as described above. RNA was aliquoted to 10 µg samples. Two independent experiments were performed and RNA isolated from the experiments was pooled for the subsequent analysis.
cDNA synthesis was carried out utilizing the superscript II cDNA-synthesis-kit (Invitrogen, Karlsruhe, Germany) according to the manufacturer's instructions. An initial amount of 10 µg RNA was used for cDNA synthesis. The cDNA labelling, microarray hybridization, and washing of the arrays was executed as recommended by the array user guide (Gene Expression Analysis v 2.0, NimbleGen^{®}). The microarrays were stored dry and in the dark until scanning.

### Scanning, quality control and interpretation of the microarrays

Scanning of microarrays and subsequent feature extraction was done with the DNA Microarray Scanner (Agilent) at a resolution of 5 µm and NimbleScan™ version 2.4 software, respectively. First, all obtained *Porphyromonas*-related data files were corrected for background intensity by comparison to the average fluorescence intensity of the negative controls. Second, all obtained *Porphyromonas*-related data files were checked for inhomogeneous hybridization results among every set of probes. Replicate spots with more than a two-fold aberration from the intensity of the other two replicates were excluded from analysis. In the case of more than a two-fold aberration between all three replicates of a single probe, all replicates of this probe set were excluded. Data were then analyzed by GeneSpring GX version 10 (Agilent technologies)

### 7.1.8 Hemagglutination assay with P. gingivalis

Colonies of solid medium-grown *P. gingivalis* (3- to 5-day cultures) were harvested and suspended in PBS, pH 7.4. 150 µl of the bacterial suspension (OD₆₆₀ 2.0) were mixed with 150 µl MF-containing solution and incubated at 37°C / 30 min. Bacteria were washed (6.000 × g, 5 min), resuspended and serially diluted in 96-well round bottom microtiter plates with PBS. A 50 µl aliquot of each dilution was mixed with 50 µl of human erythrocyte suspension (2% in PBS) and incubated for 3 h at RT. Assays were carried out in duplicate and were repeated at least three times in independent experiments. Evaluation was performed by using an UV reader (Sunrise, Tecan Austria GmbH, Salzburg, Austria) and MicroWin® software.

### 7.1.9 Gingipain assay

Bacteria from liquid overnight culture were washed, resuspended in PBS and adjusted to OD₆₆₀ of 1.0. 1 ml of this suspension was mixed with 10, 50 or 100 µg/ml of MF and incubated over 1, 10 or 30 min at 37°C. Bacteria were washed, resuspended in PBS with 1 mM L-cysteine and adusted to OD₆₆₀ of 0.26. The fluorescent protease substrates α*N*-benzoyl-L-arginine-7-amido-4-methylcoumarin and *t*-butyloxycarboyl-Val-Leu-Lys-7-amido-4-methylcoumarin (both Sigma-Aldrich, Taufkirchen, Germany) were used to determine Arg-X and Lys-X specific activities, respectively. Arg-X activity was assayed in 100 µl of PBS containing 1 mM L-cysteine, 100 µM substrate and 5 µl of bacterial suspension. Lys-X activity was assayed in 100 µl of PBS containing 1 mM L-cysteine, 10 µM substrate and 50 µl bacterial suspension. Released 7-amido-4-methylcoumarin was measured with a fluorimeter (Ascent FL, Thermoscientific, Waltham, USA) using its kinetic measurement software program over a period of 10 minutes (wave length settings: excitation 355 nm, emission 460 nm). Assays were carried out in duplicate and were repeated at least in three independent experiments. Protease activities were related to the untreated control.

### 7.1.10 Quantitation of IL-6 from KB cells

KB-cells (8 × 10⁵ cells, 70 % confluence) were cultured in 96-flat bottom microtiter well plates. 17 h prior to the experiment, the cells were washed with PBS and incubated in EMEM with 8% SerEx® (PAA Laboratories, Cölbe, Germany) and 50 µg/ml gentamicin. Treatment of KB cells started by washing them with 100 µl PBS per well and adding MF (100 µg/ml in EMEM) for 2 h / 37°C. Supernantants were collected in a sterile 96-well microtiter plate and the cell monolayers were washed with PBS. *P. gingivalis* grown to late exponential phase were resuspended in EMEM and added to KB cells at a BCR of 5:1 and 100:1 respectively. Cell culture plates were centrifuged (200 × g, 5 min) followed by incubation at 37°C / 2 h. After incubation, the culture media were collected. Cells were washed three times with 150 µl PBS/well and incubated for further 17 h at 37°C in KB cell culture medium (see above). After this incubation period, the supernatants were again collected and stored at -80°C until analyzed using Human ELISA Set IL-6 (ImmunoTools, Friesoythe, Germany) according to the manufacturer's protocol. Data are expressed as pg/ml as mean from three separate values from four independent experiments

### 7.11 Components used in the additional experiments

**Table 1: Primers and probes used for gene expression analysis in P. gingivalis. F: forward primer; R: reverse primer; P: probe;**

| mRNA target | oligonucleotides (5'→3') | |
|---|---|---|
| 16S-rRNA | F | TAACAGTTTTCGCTGAGGA |
| | R | TGTAAGGGCCGTGTTGAATTGA |
| | P | GTGAGGAAGGTGTGGA |
| *kpg* | F | GTGTATGTTGGGCTATCT |
| | R | TCCTTACTGCCACCCCTTT |
| | P | TGGAGGGAAGAGGAGT |
| *rpgA* | F | GCAGGATGAGATGAACGAAA |
| | R | GTGACCACCGAAAGTA |
| | P | AAACACCCGAACAACA |
| *fimA* | F | ACGAATAACCCAGAGAAT |
| | R | CTGACCAACGAGAACCCA |
| | P | TCACAGAGTCTGCTCAC |

**Table 2: Assay ID's for primers and probes for gene expression analysis in KB cells, synthesized by ABI, Darmstadt, Germany.**

| genes | Assay ID |
|---|---|
| IL-1β | Hs01555413_ml |
| IL-6 | Hs99999032_ml |
| IL-8 | Hs01567913_g1 |
| COX-2 | Hs01573472_g1 |
| TNF-α | Hs00174128_m1 |
| 18S rRNA | Hs99999901_s1 |

**Table 3: Changes in mRNA abundancies of P. gingivalis ATCC 33277 genes after 6 h exposure to MF as assessed by NimbleChip™ microarray hybridization experiments. Bacterial cells treated with MF (100 µg/ml) were compared to untreated cells. Data from two independent experiments were combined. Only messages with expression changes > 20-fold are shown.**

| **Corresponding Protein** | **Classification** | **Inhibition (-) Stimulation (+)** | **Fold change** |
|---|---|---|---|
| Acyl carrier protein | Biosynthesis, lipid metabolism | - | 30.7 |
| ISPg1, transposase | DNA replication | - | 21.3 |
| ISPg1, transposase | DNA replication | - | 25.7 |
| ISPg1, transposase, internal deletion | DNA replication | - | 31.1 |
| Immunoreactive 42 kDa antigen PG33 | Energy metabolism | - | 23.0 |
| RNA polymerase sigma-70, ECF subfamily | Transcription | - | 21.6 |
| Ribosomal protein S 13 | Ribosomal translation, ribosom structure | - | 22.4 |
| Ribosomal Protein L10 | Ribosomal translation, ribosom structure | - | 32.3 |
| Immunoreactive 43 kDa antigen P32 | Cell wall biosynthesis | - | 26,5 |
| Ferredoxin, 4Fe-4S | General functionality, energy metabolism | - | 20.3 |
| Hemagglutinie protein HagE | Cell adhesion, virulence | - | 19.8 |
| Hemagglutinin protein HagA | Cell adhesion, virulence | - | 27.6 |

### 7.2. Results of the additional experiments

### 7.2.1 M. flabellifolia extract MF inhibits adhesion of P. gingivalis to KB cells

From the aerial parts of *Myrothamnus flabelifolia* an ethanol/water extract MF was prepared with a phytochemical composition and fingerprinting as recently described (Engelhard et al, 2007; Anke et al., 2008). The influence of MF on the adhesion of *P. gingivalis* to human epithelial mucosa cells (KB cell line) was analyzed quantitatively by a flow cytometric assay. In principle, FITC-labelled bacteria were coincubated with a monolayer of KB cells. Non-adhering bacteria were removed by washing. After trypsin treatment suspended cells with adhering and labelled bacteria were quantified by FACS. Measured values were related to the adhesion determined in the untreated control group. MF between 0.1 and 1 mg/ml evoked dose-dependent inhibition of bacterial epithelial cell attachment (Fig. 1).

In order to investigate whether MF influenced the bacterial cell surface and/or ligand properties on the eukaryotic cell membrane, three different incubation variants were performed, namely pretreatment of bacteria, pretreatment of KB cells and coincubation of bacteria and KB cells in the presence of MF (0.1 mg/ml). Data obtained clearly indicated that preincubation of *P. gingivalis* strongly reduced bacterial binding to KB cells, while pretreatment of eukaryotic cells showed only moderate effects (compare Fig. 2). Cotreatment led to considerable effects, but less than those obtained by preincubation of the bacteria. From these data it is concluded that MF mainly influences the bacterial surface.

### 7.2.2 MF (100 µg/ml) is not toxic for P. gingivalis

For further evaluation of the mode of action a potential direct cytotoxicity of MF (0.4 mg/ml) for *P. gingivalis* was tested by agar diffusion assay over 5 days, employing amoxicillin as positive control. Bacterial growth was not influenced by MF (data not shown). In addition, the influence of MF (1 to 1000 µg) on *P. gingivalis* grown in liquid culture was investigated by determination of cellular viability by MTT test (Moosman 1983). Exposure to MF at < 150 µg/ml over 30 min (the contact time used for the anti-adhesion assay) did not influence viability of *P. gingivalis.* EC₅₀ values for a 24 h incubation were determined as 114 µg/ml. From these data cytotoxic effects of MF against the bacteria can be excluded under the assay conditions. i.e. even a higher concentration than the one given for MF1 in Table Thus the extract MF is even tolerated at a higher concentration (< 150 µg/ml versus < 125 µg/ml) than the extracts MF1, MF2 and MF3 as shown above in the table on page 9. Therefore, the antiadhesive effect observed is probably due to interaction with fimbriae or outer membrane proteins (OMP).

### 7.2.3 MF inhibits hemagglutination and gingipains

In order to investigate the influence of MF on the major adhesin OMPs, hemagglutinin, arginin- and lysine-gingipain activities were monitored. An inhibiting activity of MF against bacterial hemagglutinin was confirmed by hemagglutination assay. *P. gingivalis* were pretreated for 30 min with different concentrations of MF (1 to 1000 µg/ml) and incubated after a serial dilution together with human erythrocytes. MF-treated bacteria displayed a substantial inhibition of hemagglutination (data not shown).

For differentiation of MF effects on the Arg- and Lys-gingipain activities, specific fluorescent-labelled peptide substrates were used for protease assays, namely αBAAM (αN-benzoyl-L-arginin-7-amino-4-methylcoumarin) for Arg-gingipain and t-boc-VLA (t-butyloxycarboyl-val-leu-lys-7-amino-4-methylcoumarin) for Lys-gingipain (Potempa et al. 2007). The tripeptide leupeptide (100 µM), a specific inhibitor of arg-gingipain, served as positive control (Baba et al, 2001). MF inhibited Arg-gingipain in a concentration-dependent manner (Fig. 3). After 1 min contact time, about 50 % inhibition was observed at 1 µg/ml. Prolonged incubation time did not further increase the anti-protease effects. MF at 50 µg/ml reduced Arg-gingipain by 20 to 30%, 100 µg/ml by about 20%. MF did also inhibit Lys-gingipain, but to a lesser extent. These findings indicate MF to be a potent gingipain inhibitor, predominantly because of its activity against Arg-gingipain.

### 7.2.4. Influence of MF on gene expression of major adhesins

The question arose if the inhibition of gingipains would lead to changes in gene expression of the respective adhesins potentially via a cellular feed back mechanism, resulting in an increased expression of surface structures with binding affinity to host ligands. Therefore, RT PCR studies were performed on the influence of MF on the expression of *rgpA* for Arg-gingipain, *kgp* for Lys-gingipain and *fimA* for fimbrillin (Fig. 4). Expression rates of the respective genes were constant in *P. gingivalis* liquid cultures over incubation periods from 2 to 12 h, as monitored by the corresponding ΔCT values. Treatment of the bacteria with MF (10, 100 µg/ml) influenced the gene expression moderately: While *kgp* expression was slightly reduced, a significant, about 3- to 4-fold increase for *rgpA* and *fimA* was observed. In conclusion, the data document *MF* inhibition of bacterial adhesin functions; as a response, the bacteria seem to compensate this inhibition by overexpression of the respective OMPs.

### 7.2.5 Influence of MF on genome-wide gene expression by microarray analysis

For a holistic investigation on the influence of *MF* on *P. gingivalis* genes, a transcriptome microarray was performed. *P. gingivalis* were treated in liquid culture for 6 h with MF (100 µg/ml). Two independent experiments were performed, and pooled RNA was used for microarray, investigating the expression of 2015 genes. The Cy3- and Cy5- labelled cDNAs from MF-exposed and not exposed *P. gingivalis* were hybridized to the immobilized oligonucleotides. Levels of gene expression from treated and untreated bacterial cells were compared by the ratio of fluorescence intensities (Table 3). In total, changes in transcript abundancies of 550 genes were monitored when using a threshold of 3-fold difference; judged by transcript amounts 308 genes appeared as down- and 242 genes as up-regulated. A listing of genes influenced by MF is provided in the Supplemental Information. Generally, the majority of influenced genes showed 3- to 4-fold changes. Drastic differences in transcript abundancies with values of ≥ 20-fold were found for 12 genes (Tab. 3).
The microarray data were in good agreement with RT-PCR results for the expression of adhesin genes in presence / absence of MF. According to the microarray data, the fimbrillin message was 3.5-fold increased, while the mRNA for the arginin-specific cysteine proteinase was 12.6-fold decreased (see Supplemental Information). Messages from 3 hemagglutination genes were less abundant (hemagglutinin-related protein: -7.4, hemagglutinin protein HagE: -12.6, hemagglutinin protein HagA: -27.6).
Of note, mRNAs from gene clusters responsible for DNA transcription and translation control were 20- to 30-fold reduced; especially messages from transposase genes and genes encoding translation-related enzymes with influence on ribosomal activity were considerably less concentrated in the presence of MF.

### 7.2.6 Cytoprotective effects of MF against P. gingivalis induced cytokine induction

A prominent activity of *P. gingivalis* infection in KB cells is the induction of cellular inflammation response. Using gene expression studies for monitoring TNFα, IL-1β, IL-6, IL-8 and COX-2 as typical inflammation markers, treatment of KB cells with bacteria (bacteria cell ratio BCR 5:1) for 6 hours resulted in significant, about 5-fold increase of IL-1β, IL-6, IL-8 and TNFα expression, while COX-2 was not influenced (Fig. 5A). When KB cells were pretreated for 2 hours with MF (10 and 100 µg/ml), followed by removal of the incubation medium, washing of the cells and treatment with *P. gingivalis* (BCR 5:1) this pretreatment resulted in clearly decreased gene expression for cytokines, which were significant lower than in control cells not exposed to MF (Fig. 5B).
To investigate the influence of MF on the expression of IL-6 on protein level, KB cells were preincubated for 2 h with MF (100 µg/ml). The extract was removed and the cells were incubated with *P. gingivalis* (BCR 5:1 or 100:1 for 2 h). After removal of the bacteria from the cell culture IL-6 amounts were quantified from the culture supernatant ("2 h measurement"). Cells were further incubated in bacteria- and MF-free media and IL-6 concentrations were additionally determined 17 hours after infection. Differences were calculated against the respective values obtained from non-exposed cells. As shown in Fig. 6, the cytokine formation in untreated control groups was < 15 pg/ml after 2 h for cells infected with BCR 5:1 and 100:1. Similar low concentrations were measured for the MF treated cells. This indicates that the IL-6 formation in KB cells is a rather slow process. 17 h after treatment IL-6 concentrations increased by about 10-fold within the untreated control groups. Preincubation of the KB cells with MF resulted in significant lower IL-6 amounts, indicating a cytoprotective effect of MF against the cells.

### 7.3 Summary of supplementary findings of the additional experiments

The supplementary findings further support the concept that polyphenol enriched plant derived extract can strongly interfere with adhesion of *P. gingivalis* to epithelial KB cells under *in vitro* conditions and show that this is due to inhibition of Arg-gingipains and hemaglutinin. As shown the polyphenol-enriched MF extract exhibits inhibition of *P. gingivalis* adhesion to epithelial cells, with strong influence against Arg-gingipain and less inhibition of the respective lysine-protease, indicating a certain degree of selectivity of the polyphenols. Also the hemagglutinin function is inhibited to a minor extent. The main target of the extract appears to be the bacterial surface, but also preincubation of KB cells with the extract led to a slightly reduced *P. gingivalis* attachment. This could be due to a binding of the multivalent polyphenols to surface proteins of KB cells, changing the structure of potential ligands for the bacterial binding.

Additionally, cytoprotective effects of the extract against inflammation related parameters of KB cells were shown, i.e. preincubation of the KB cells with extract and subsequent exposure of the cells to *P. gingivalis* even in the absence of the extract substantially reduced the expression of inflammation-associated cytokines, either by decreasing the bacterial load or by down regulation of the cellular response. Therefore the hypothesis, that the extract from *M. flabellifolia* can be used for prophylactic treatment against *P. gingivalis* infection was proven.

It seems interesting that inhibition of the Arg-gingipain proteases leads to a feed back mechanism of increased gen expression for *rgpA,* which indicates that the gingipain activity is controlled by bacterial cellular programs. Moreover a slightly increased gene expression of *fimA* was proven, which could be due to an influence of MF on the fimbriae. What was clearly seen is the reduced activation of inflammatory cytokines in infected KB cells. This may be due to the inactivation or inhibition of fimbriae by MF.

Due to the fact that *P. gingivalis* cell adhesion predominantly relies on the functions of gingipain proteases, the use of antiadhesive compounds influencing protein-protein interactions should be favoured. Here the use of polyphenol mixtures should be favored due to two reasons: i) adstringent affinity of proanthocyanidins to proteins is dependent on structural features, ii) high molecular compounds, especially longer oligomeric or polymeric proanthocyanidins, can exert strong interaction, but have a low solubility. In contrast, low molecular derivatives are perfectly soluble under aqueous conditions, acting very fast against proteins, but have a low astringency and do not adhere over longer time intervals to proteins. Complex mixtures of polyphenols with different clusters of polymerisation and different substructures also have the advantage of influencing their own solubility, probably by formation of charge-transfer complexes between the different molecules.

It should be noted that a major problem of the *M. flabellifolia* extract for clinical use is the safety issue: at higher doses the extract can exert significant decrease of KB cell vitality and toxicity against *P. gingivalis.* However, the additional results demonstrate that concentrations below 150 µg/ml only influence bacterial cell surface structures without exerting negative effects on host cell physiology. Therefore, potential clinical use of such extracts should be restricted to multiple low dose applications.

### References

Shi J et al. "Polyphenolics in grape seeds-biochemistry and functionality ", J Med Food. 2003 Winter; 6(4):291-9.
Anke, J., Petereit, F., Engelhardt, C. & Hensel, A. (2008) Procyanidins from Myrothamnus flabellifolia Welw. Natural Product Research 22, 1243-54.

### Figures

Fig. 1: Extraction scheme from *Myrothamnus flabellifolia* Welw. for the example of *M. Flabellifolia.* MF = *Myrothamnus flabellifolia.* This scheme is applicable similarly for Rumex. After shaking with ethyl acetate, the aqueous phase (MF3/RA3) contains mainly higher-molecular proanthocyanidin fractions (≥ tetramers). As shown by the applicant's investigations, these are in particular hepta- to octamers, which owing to solubility can only be in the aqueous phase and, because of their size, cannot pass through cellular barriers. This fraction is therefore, as shown later, less strongly antibacterial or cytotoxic. At the same time their action is restricted to the cell exterior, i.e. the region which are there are adhesins.
Fig. 2: Results of a cell adhesion assay of *P. gingivalis* on KB cells by means of a FACS-assay after preincubation of the KB cells with the test substances (90 minutes, 100 µg/mL). Untreated control = 100%. (n=3-5). It can be seen in particular that cells treated with the extracts RA2 and MF1 were colonized less strongly by *P. gingivalis.*
Fig. 3: Results of the FACS-assay with coincubation of the KB cells with the test substances and *P. gingivalis* (n=3-5) at different extract concentrations. (90 minutes, 100 / 50/10 µg/mL). It shows the adhesion of *P. gingivalis* on the KB cells in [%]. Untreated control = 100%.
Fig. 4: Results of the effects of the test substances [10 µg/mL] on biofilm formation of *Streptococcus mutans),* 3-day incubation, changing of the medium (with test substance) every 24 hours). Untreated control = 100%. (n=3-6). The extracts MF2, MF3 and RA1 prove especially effective.
Fig. 5: Investigation of the direct cytotoxicity of various extracts of *Myrothamnus flabellifolia* Welw. against KB cells after 24-hour incubation by means of MTT-test. Untreated control = 100% (n=4). It can be seen that the extracts MF1, MF2 and MF4 have relatively higher cytotoxicity than extract MF3. The latter contains preferentially higher-meric (≥ tetrameric) proanthocyanidins, which because of their higher molecular weight have a lower bioavailability and therefore as a rule do not penetrate into the cells.
Fig. 6: Investigation of the direct cytotoxicity of various extracts of *Rumex acetosa* L. against KB cells after 24-hour incubation by means of MTT-test. Untreated control = 100% (n=4). It can be seen that the extracts RA1, RA2 and RA4 have a relatively higher cytotoxicity than extract RA3. The latter contains preferentially higher-meric (≥ tetrameric) proanthocyanidins, which owing to their higher molecular weight have a lower bioavailability and therefore as a rule do not penetrate into the cells.

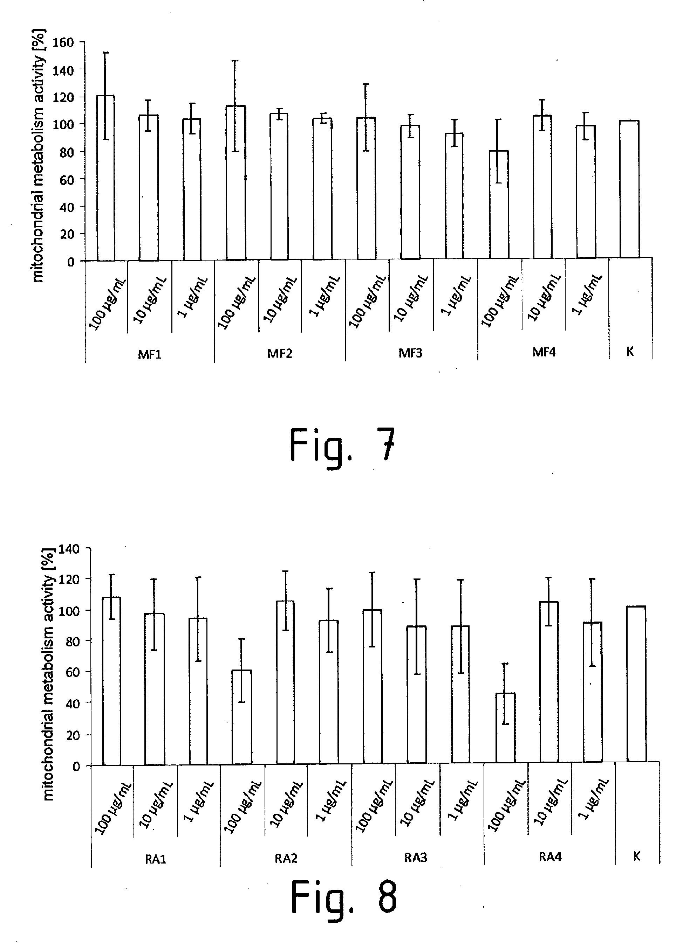

Fig. 7: Investigation of the direct cytotoxicity of various extracts of *Myrothamnus flabellifolia* Welw. against KB cells after 6-hour incubation by means of MTT-test. Untreated control = 100% (n=4). It can be seen that the differences between the various extracts are much less pronounced, owing to the considerably shorter incubation time (1/4) in comparison with Fig. 5.
Fig. 8: Investigation of the direct cytotoxicity of various extracts of *Rumex acetosa* L.. Against KB cells after 6-hour incubation by means of the MTT-test. Untreated control = 100% (n=4). It can be seen that the differences between the various extracts are very much less pronounced, owing to the considerably shorter incubation time (1/4) in comparison with Fig. 7; however, it can be seen that extracts RA2 and RA4 have a quite high cytotoxicity at sufficiently high concentrations even with this relatively short incubation time.
Fig. 9: Investigation of the direct cytotoxicity of various extracts of *Rhododendron ferrugineum* L. against KB cells after 24-hour incubation by means of the MTT-test. Untreated control = 100% (n=4). It can be seen that the differences between the various extracts are very small, because of the short incubation time.
Fig. 10: Bactericidal action of *Myrothamnus flabellifolia* extracts on *S. mutans* after an incubation time of 24 hours. Measurement by the MTT-test. Untreated control = 100% (n=4). It can be seen that extract MF4 has the strongest bactericidal action. This indicates that in this extract there are relatively lower-meric (≤ tetrameric) proanthocyanidins, which because of their low molecular weight have a relatively good bioavailability, thus can penetrate into the cells and exert bactericidal action there. Moreover, it can be seen that with an incubation time of 24 h in the case of MF4 the bactericidal action already starts at a concentration of 125 µg/ml, whereas with the other extracts the bactericidal action only starts at a concentration of 250 µg/ml.
Fig. 11: Bactericidal action of *Rumex acetosa* extracts on *S. mutans* after an incubation time of 24 hours. Measurement by the MTT-test. Untreated control = 100% (n=4). It can be seen that extract RA4 has the strongest bactericidal action. This indicates that in this extract there are relatively lower-meric (≤ tetrameric) proanthocyanidins, which due to their low molecular weight have a relatively good bioavailability, thus can penetrate into the cells and exert bactericidal action there. Moreover, it can be seen that with an incubation time of 24 h in the case of MF4 the bactericidal action already starts at a concentration of 125 µg/ml, whereas with the other extracts the bactericidal action only starts at a concentration of 250 µg/ml.
Fig. 12: Bactericidal action of *Myrothamnus flabellifolia* extracts on *P. gingivalis* after an incubation time of 30 minutes. Measurement by the MTT-test. Untreated control = 100% (n=3). Owing to the shorter incubation time, the differences between the individual extracts are less pronounced, and generally much higher concentrations are required to exert a bactericidal action. Thus, with this incubation time, concentrations of 125 µg/ml or 250 µg/ml still have no bactericidal action; however, with correspondingly high concentration, MF4 is again the fraction with the highest bactericidal action.

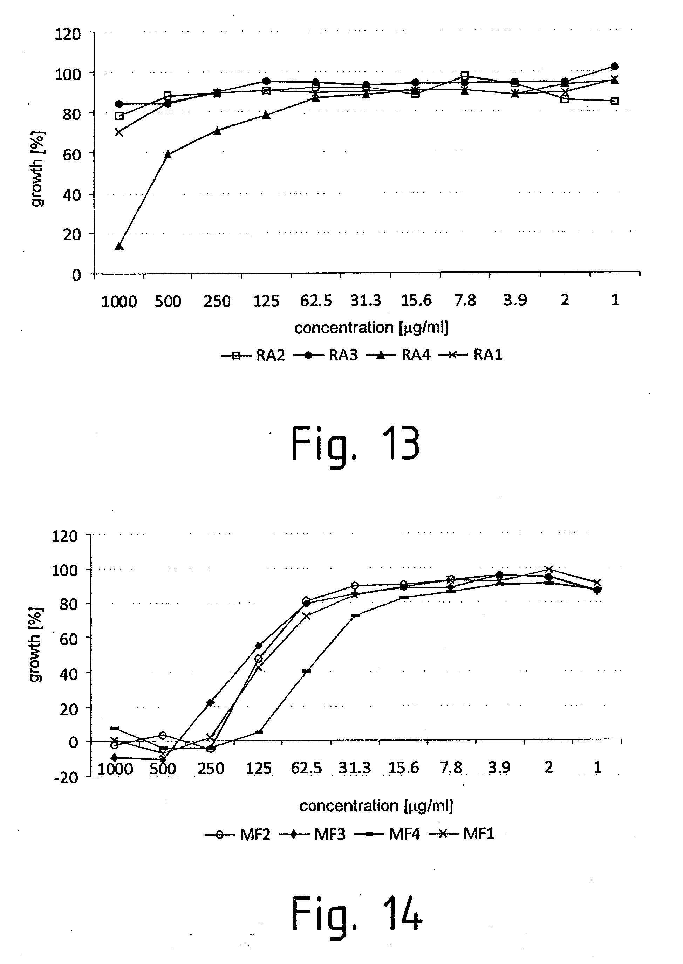

Fig. 13: Bactericidal action of *Rumex acetosa* extracts on *P. gingivalis* after an incubation time of 30 minutes. Measurement by the MTT-test. Untreated control = 100% (n=3). Owing to the shorter incubation time, the differences between the individual extracts are less pronounced, and generally much higher concentrations are required to exert a bactericidal action. Thus, with this incubation time, concentrations of 125 µg/ml or 250 µg/ml still have no bactericidal action; however, with a correspondingly high concentration, RA4 is again the fraction with the highest bactericidal action.
Fig. 14: Bactericidal action of *Myrothamnus flabellifolia* extracts on P. *gingivalis* after an incubation time of 24 hours. Measurement by the MTT-test. Untreated control = 100% (n=4). It can again be seen that MF4 already exerts a bactericidal action at lower concentrations (62.5 µg/ml) than the other extracts, which only do so at higher concentrations (125 µg/ml).
Fig. 15: Bactericidal action of *Rumex acetosa* extracts on *P. gingivalis* after an incubation time of 24 hours. Measurement by the MTT-test. Untreated control = 100% (n=4). Once again it can be seen that RA4 already exerts a bactericidal action at lower concentrations (62.5 µg/ml) than the other extracts, which only do so at higher concentrations (250 µg/ml).
Fig. 16: Influence on gene expression of various inflammation markers (interleukins IL-1b, IL-6 and IL-8 and tumor necrosis factor TNF-α) in KB cells by 2-hour preincubation with 100 µg/mL RA2 and subsequent two-hour incubation with *P. gingivalis* (BCR 5:1). Untreated control = 1 (n = 2). It can be seen that said preincubation suppresses gene expression of the said inflammation markers considerably in comparison with untreated controls.
Fig. 17: Influence on gene expression of various inflammation marker (interleukin IL-8 and tumor necrosis factor TNF-α) in KB cells by 2-hour preincubation with 10 µg/mL MF1 and subsequent two-hour incubation with *P. gingivalis* (BCR 5:1). Untreated control = 1 (n = 2). It can be seen that said preincubation suppresses gene expression of said inflammation markers considerably in comparison with untreated controls.
Fig. 18: Mean adhesion values of FITC-labelled MF-exposed *Porphyromonas gingivalis* to KB cells. The bacteria were preincubated for 30 min at 37°C with different concentrations of MF. Data are related to the untreated control (= 100%). Data are mean values ± SD (n ≥ 3); ***p* < 0.01; ****p* < 0.005. This experiment was carried out with the additional *Myrothamnus flabellifolia extract "MF".*

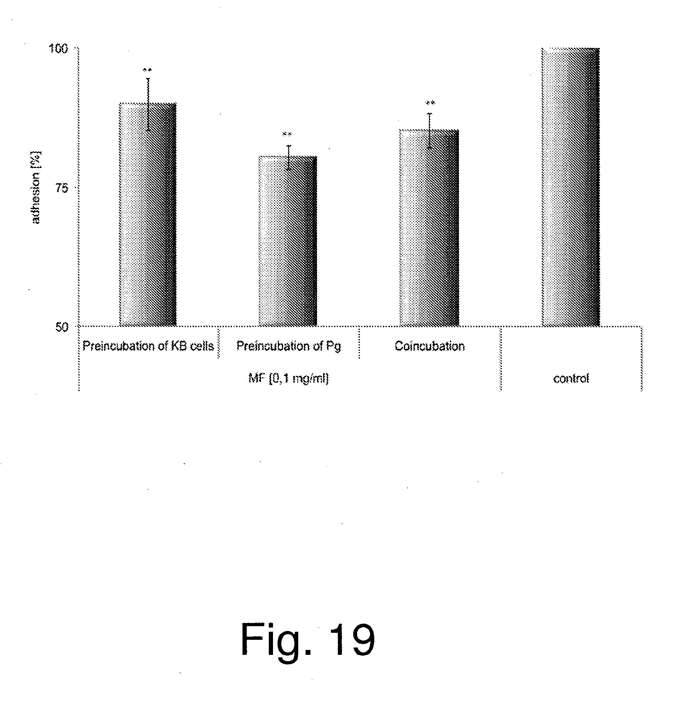

Fig. 19: Inhibitory effects of MF (0.1 mg/ml) on *P. gingivalis* adhesion to KB-cells under different incubation variants: Experiments were performed after preincubation of KB cells, preincubation of *P. gingivalis,* and after coincubation of KB cells together with *P. gingivalis* and MF. Data are mean ± SD (n ≥ 3); ** p < 0.01. This experiment was carried out with the additional *Myrothamnus flabellifolia extract "MF ".*

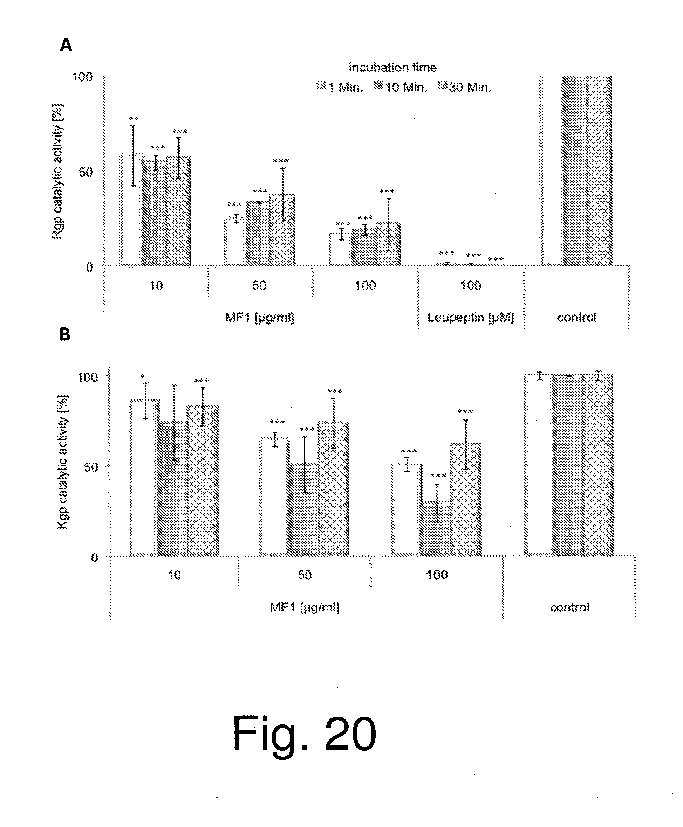

Fig. 20: Influence of MF (10, 50 and 100 µg/ml) on Arg-gingipain (A) and Lys-gingipain (B) activity of *P*. *gingivalis* within protease assay after incubation of bacterial over 1, 10 or 30 minutes. Positive control: leupeptin, negative control: untreated bacteria. Data are mean ± SD (n = 4). * p < 0.05; **: p < 0.01; ***: p < 0.005. This experiment was carried out with the additional *Myrothamnus flabellifolia extracts "MF".*

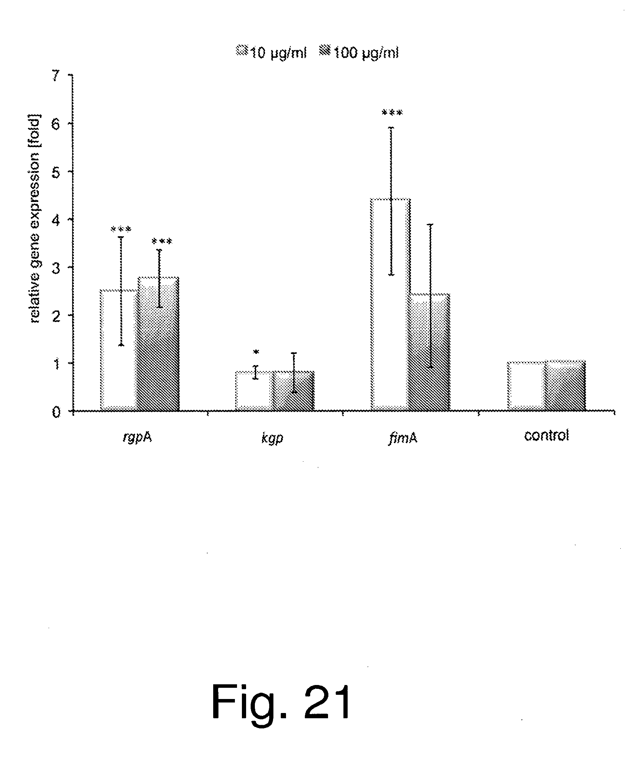

Fig. 21:Influence of MF on relative gene expression from *P. gingivalis* on *rgpA*, encoding for Arg-gingipain, *kgp,* encoding for Lys-gingipain and *fimA,* encoding for fimbrillin after 6 hours incubation with 10 and 100 µg/ml MF. Data are mean ± SD (n = 3);* p < 0.05; *** p < 0.005. This experiment was carried out with the additional *Myrothamnus flabellifolia extract* "*MF*".

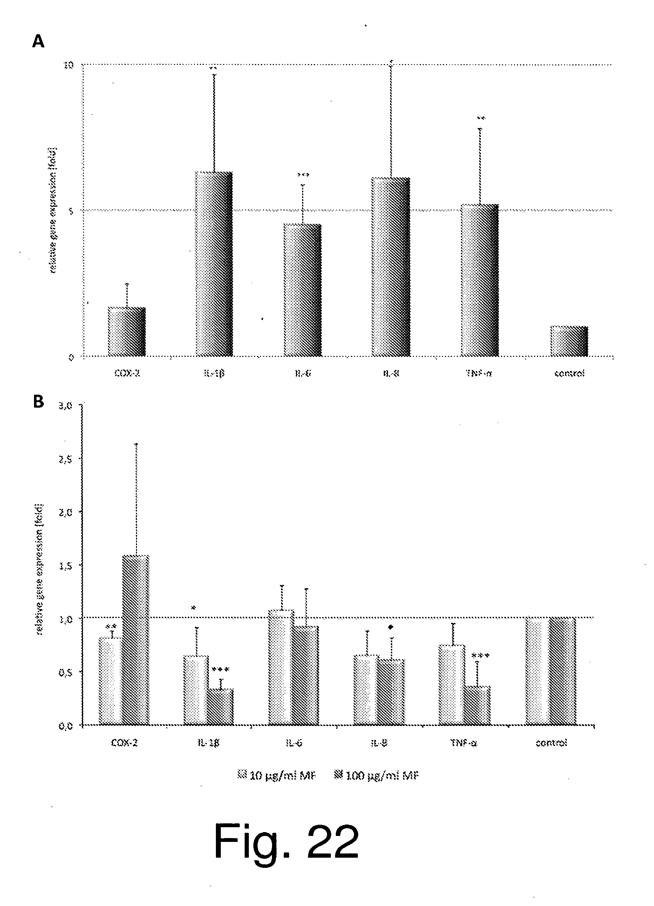

Fig. 22: Cytoprotective influence of MF on inflammation parameters of KB cells after exposure to *P. gingivalis.* This experiment was carried out with the additional *Myrothamnus flabellifolia extract "MF".*
   A: Influence of P. *gingivalis* exposure (BCR 5:1, 2 h) on relative gene expression (RT PCR) of COX-2, IL-1β, IL-6, IL-8 and TNFα.
   B: Influence of MF pretreatment of KB cells (2 h; 10, 100 µg/ml) prior to exposure to *P. gingivalis* (BCR 5:1, 2 h) on relative gene expression (RT PCR) of COX-2, IL-1β, IL-6, IL-8 and TNFα.
   Data are mean ± SD (n = 4 independent experiments) and related to untreated control groups * p < 0.05; ** p < 0.01; *** p < 0.005.

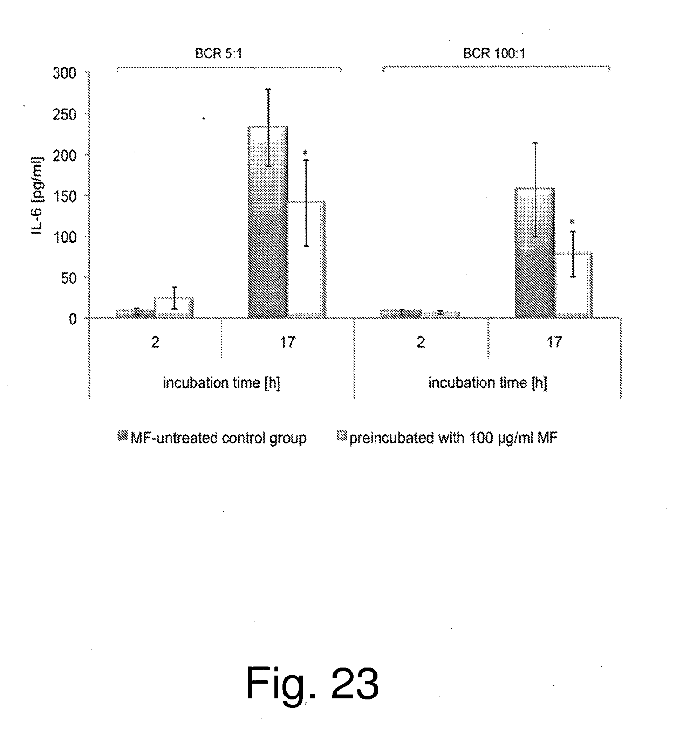

Fig. 23:IL-6 concentration in the culture supernatant of KB cells as determined by ELISA: KB cells were preincubated for 2 h with MF (10, 100 µg/ml) prior to exposure to *P. gingivalis* (BCR 5:1 and 100:1). Coincubation time with bacteria was 2 h, further incubation of the cells after removal of bacteria fro 17 h in total. IL-6 was determined 2 and 17 h after addition of the bacteria to the KB cells. Data are mean ± SD (n = 4) and related to the untreated control groups; * p < 0.05. This experiment was carried out with the additional *Myrothamnus flabellifolia extract "MF".*

The following tables show selected proanthocyanidins from *Rumex* and *Myrothamnus* (unclaimed), which can be used.

**Proanthocyanidins from Myrothamnus flabelifola**

| **Structural formula** | **No.** | **R₁** | **R₂** | **R₃** | **R₄** | **Name** |
|---|---|---|---|---|---|---|
| 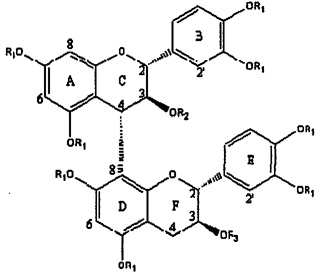 | M4 | H | | | | catechol-(4a→8)-catechol (procyanidin B3) |
| | M4a | Ac | | | | catechol-(4a→8)-catechol peracetate |
| 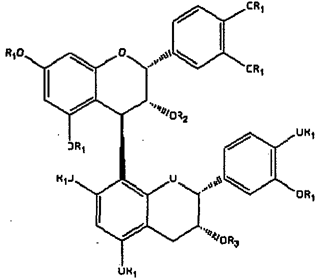 | M8 | H | H | G | | epicatechol-(4β→8)-epicatechol-3-O-gallate |
| | M8a | Ac | Ac | G | | |
| | M11 | H | G | G | | epicatechol-3-O-gallate-(4β→8)-epicatechol-3-O-gallate |
| | M11a | Ac | G | G | | |
| 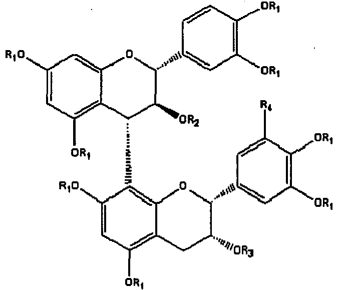 | M5 | H | H | H | H | catechol-(4a→8)-epicatechol (procyanidin B4) |
| | M5a | Ac | Ac | Ac | H | |
| | M7 | H | H | H | OH | catechol-(4α→8)-epigallocatechin |
| | M7a | Ac | Ac | Ac | OAc | |
| | M10 | H | H | G | H | catechol-(4α→8)-epicatechol-3-O-gallate |
| | M10a | Ac | Ac | G | H | |
| | M12 | H | H | G | OH | |
| | M12a | Ac | Ac | G | OAc | |
| 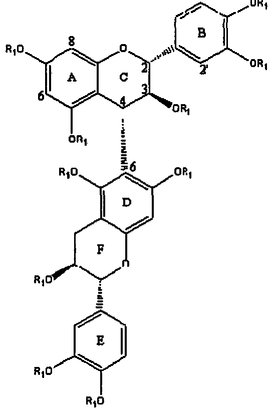 | M9 | H | | | | catechol-(4α→6)-catechol (procyanidin B6) |
| | M9a | Ac | | | | |
| 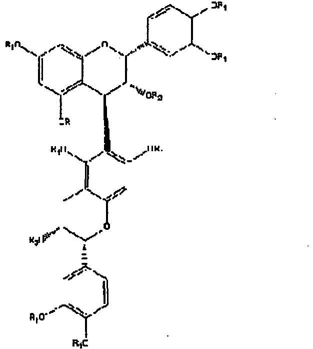 | M13 | H | G | HB | | |
| | M13a | Ac | G | HB | | |
| | M14 | H | G | G | | |
| | M14a | Ac | G | G | | |
| 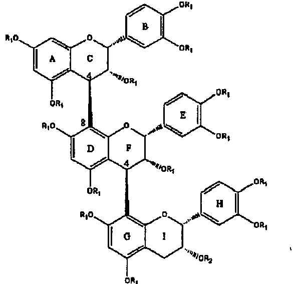 | M15 | H | G | | | |
| | M15a | Ac | G | | | |
| 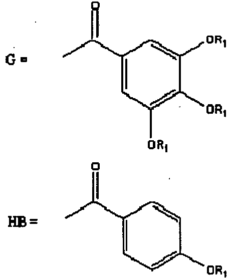 | | | | | | |

**Proanthocyanidins from Rumex**

| **Structural formula** | **No.** | **Config. on C-2 and C-3** | | **R₁** | **R₂** | | | **Name (monomer)** |
|---|---|---|---|---|---|---|---|---|
| 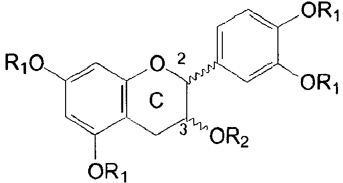 | | | | | | | | |
| | R1 | 2R, 3S | | H | H | | | |
| | R1 | 2S, 3R | | H | H | | | |
| | R1a | 2R, 3S | | Ac | Ac | | | |
| | R1a | 2S, 3R | | Ac | Ac | | | |
| | R2 | 2R, 3R | | H | H | | | |
| | R2a | 2R, 3R | | Ac | Ac | | | |
| | R3 | 2R, 3R | | H | G | | | |
| | R3a | 2R, 3R | | H | G_{Ac} | | | |
| 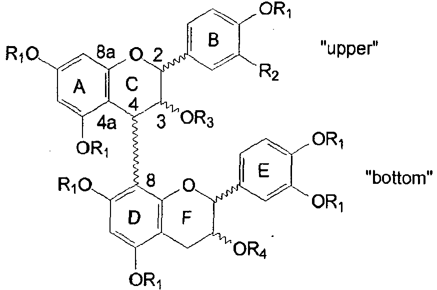 | **No** | **Config. on C-2, C-3 and C-4** | **Config. on F-2 and F-3** | R₁ | R₂ | R₃ | R₄ | **Name (dimer)** |
| | R4 | 2R, 3R, 4R (β) | 2R, 3S | H | OH | H | H | epicatechol-(4β→8)-catechol (procyanidin B) |
| | R4a | 2R, 3R, 4R (β) | 2R, 3S | Ac | OAc | Ac | Ac | |
| | R5 | 2R, 3R, 4R (β) | 2R, 3R | H | OH | H | H | epicatechol-(4β→8)-epicatechol (procyanidin B2) |
| | R5a | 2R, 3R, 4R (β) | 2R, 3R | Ac | OAc | Ac | Ac | |
| | R6 | 2R, 3S, 4S (α) | 2R, 3S | H | OH | H | H | catechol-(4α→8)-catechol (procyanidin B3) |
| | R6a | 2R, 3S, 4S (α) | 2R, 3S | Ac | OAc | Ac | Ac | |
| | R7 | 2R, 3S, 4S (α) | 2R, 3R | H | OH | H | H | catechol-(4α→8)-epicatechol (procyanidin B4) |
| | R7a | 2R, 3S, 4S (α) | 2R, 3R | Ac | OAc | Ac | Ac | |
| | R8 | 2R, 3R, 4R (β) | 2R, 3R | H | H | H | H | epiafzelechin-(4β→8)-epicatechol |
| | R8a | 2R, 3R, 4R (β) | 2R, 3R | Ac | H | Ac | Ac | |
| | R9 | 2R, 3R, 4R (β) | 2R, 3R | H | H | H | G | |
| | R9a | 2R, 3R, 4R (β) | 2R, 3R | Ac | H | Ac | G_{Ac} | |
| | R10 | 2R, 3R, 4R (β) | 2R, 3R | H | H | G | G | epiafzelechin-3-O-gallate-(4β→8)-epicatechol-3-O-gallate |
| | R10a | 2R, 3R, 4R (β) | 2R, 3R | Ac | H | G_{Ac} | G_{Ac} | |
| | R11 | 2R, 3R, 4R (β) | 2R, 3R | H | OH | H | G | epicatechol-(4β→8)-epicatechol-3-O-gallate |
| | R11a | 2R, 3R, 4R (β) | 2R, 3R | Ac | OAc | Ac | G_{Ac} | |
| | R12 | 2R, 3R, 4R (β) | 2R, 3R | H | OH | G | G | epicatechol-3-O-gallate-(4β→8)-epicatechol-3-O-gallate |
| | R12a | 2R, 3R, 4R (β) | 2R, 3R | Ac | OAc | G_{Ac} | G_{Ac} | **Name (dimer)** |
| 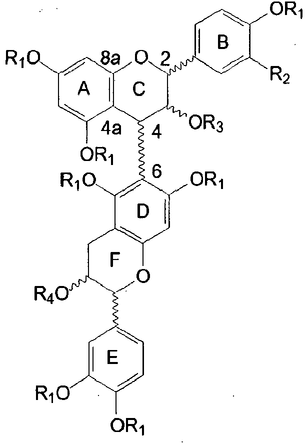 | **No** | **Config. on C-2, C-3 and C-4** | **Config. on F-2 and F-3** | **R₁** | **R₂** | **R₃** | **R₄** | |
| | R14 | 2R, 3R, 4R (β) | 2R, 3R | H | OH | H | H | epicatechol-(4β→6)-epicatechol (procyanidin B5) |
| | R14a | 2R, 3R, 4R (β) | 2R, 3R | Ac | OAc | Ac | Ac | |
| | R15 | 2R, 3R, 4R (β) | 2R, 3S | H | OH | H | H | epicatechol-(4β→6)-catechol (procyanidin B7) |
| | R15a | 2R, 3R, 4R (β) | 2R, 3S | Ac | OAc | Ac | Ac | |
| | R16 | 2R, 3R, 4R (β) | 2R, 3R | H | H | H | G | |
| | R16a | 2R, 3R, 4R (β) | 2R, 3R | Ac | H | Ac | G_{Ac} | |
| | R17 | 2R, 3R, 4R (β) | 2R, 3R | H | OH | H | G | epicatechol-(4β→6)-epicatechol-3-O-gallate |
| | R17a | 2R, 3R, 4R (β) | 2R, 3R | Ac | OAc | Ac | G_{Ac} | |
| | R18 | 2R, 3R, 4R (β) | 2R, 3R | H | OH | G | G | epicatechol-3-O-gallate-(4β→6)-epicatechol-3-O-gallate |
| | R18a | 2R, 3R, 4R (β) | 2R, 3R | Ac | OAc | G_{Ac} | G_{Ac} | |
| 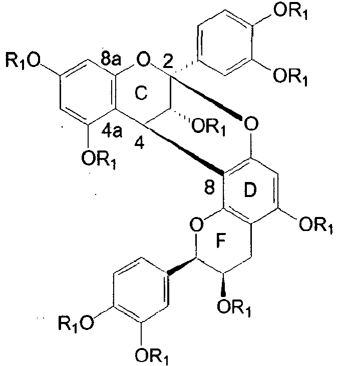 | **No** | | | **R₁** | | | | **Name (dimer)** |
| | R13 | | | H | | | | epicatechol-(2β→7, 4β→8)-epicatechol (procyanicdin A2) |
| | R13a | | | Ac | | | | |
| 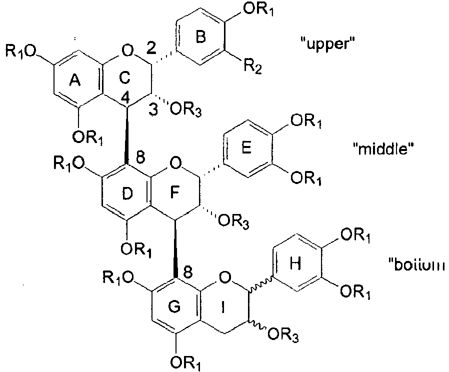 | **No** | **Config. on I-2 and I-3** | | **R₁** | **R₂** | **R₃** | | **Name (trimer)** |
| | R19 | 2R, 3R | | H | H | H | | |
| | R19a | 2R, 3R | | Ac | H | Ac | | |
| | R20 | 2R, 3R | | H | OH | H | | epicatechol-(4β→8)-epicatechol-(4β→8)-epicatechol (procyanidin C1) |
| | R20a | 2R, 3R | | Ac | OAc | Ac | | |
| | R21 | 2R, 3S | | H | OH | H | | epicatechol-(4β→8)-epicatechol-(4β →8)-catechol |
| | R21a | 2R, 3S | | Ac | OAc | Ac | | |
| | R22 | 2R, 3R | | H | OH | G | | epicatechol-3-O-gallate-(4β→8)-epicatechol-3-O-gallate-(4β→8)-epicatechol-3-O-gallate |
| | R22a | 2R, 3R | | Ac | OAc | G_{Ac} | | |
| 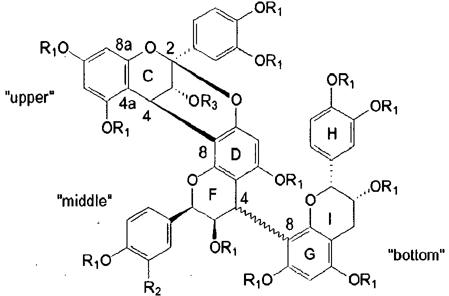 | **No** | **Config. on F-4** | | **R₁** | **R₂** | **R₃** | | **Name (trimer)** |
| | R23 | 4R (β) | | H | OH | H | | epicatechol-(2β→7, 4β→8)-epicatechol-(4β→8)-epicatechol (cinnamtannin B1) |
| | R23a | 4R (β) | | Ac | OAc | Ac | | |
| | R24 | 4S (α) | | H | H | H | | |
| | R24a | 4S (α) | | Ac | H | Ac | | |
| | R25 | 4R (β) | | H | OH | G | | |
| | R25a | 4R (β) | | Ac | OAc | G_{Ac} | | |
| 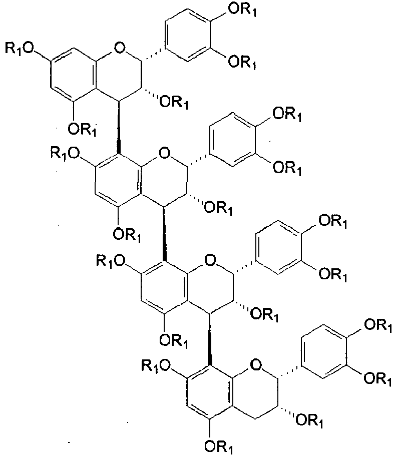 | **RNo** | | | **R₁** | **R₂** | **R₃** | | **Name (tetramer)** |
| | R26 | | | H | | | | epicatechol-(4β→8)-epicatechol-(4β→8)-epicatechol-(4β→8)-epicatechol (procyanidin D1) |
| | R26a | | | Ac | | | | |
| 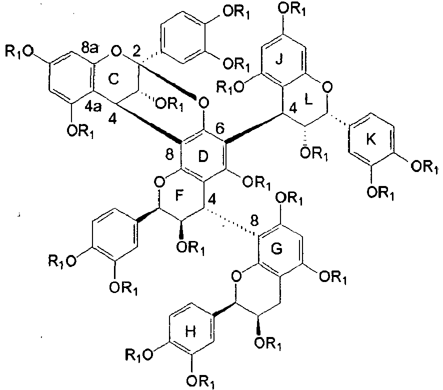 | **No** | | | **R₁** | **R₂** | **R₃** | | **Name (tetramer)** |
| | R27 | | | H | | | | epicatechol-(2β→7.4β→8)-[epicatechol-(4β→6)]-epicatechol-(4β→8)-epicatechol (parameritannin A1) |
| | R21a | | | Ac | | | | |
| 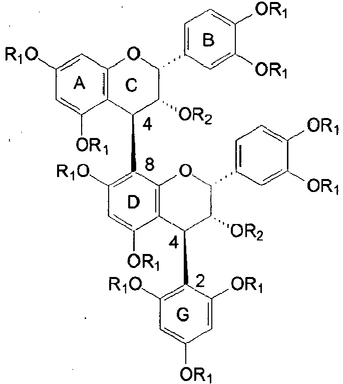 | **No** | | | **R₁** | **R₂** | **R₃** | | |
| | R29 | | | H | | | | epicatechol-(2β→7,4β→8)-epicatechol-[4β→2]-phloroglucinol |
| | R29a | | | Ac | | | | |
| 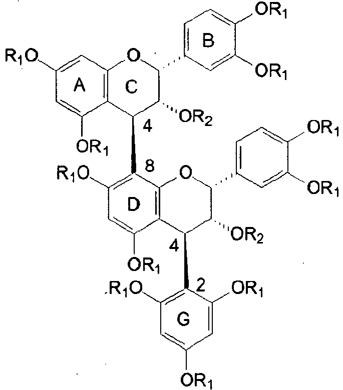 | **No** | | | **R₁** | **R₂** | | | |
| | R30 | H | | H | G | | | epicatechol-3-0-gallate-(4β→8)-epicatechol-3-*O-*gallate-(4β→2)-phloroglucinol |
| | R30a | Ac | | Ac | G_{Ac} | | | |

## Claims

1. Composition containing at least one oligomeric or polymeric flavan-3-ol unit (proanthocyanidin) from a plant *Rumex acetosa* extract, for use in prevention or treatment of gingivitis, periodontitis, caries, ulcer of the oral mucosa (aphtha), or gingivostomatitis wherein the composition prevents the adhesion of at least one bacterium on mucosa, tongue, palate, gingiva, gingival pocket or tooth surface in a exposure time that does not exceed 10 minutes.

2. The composition for use according to claim 1, **characterized in that** the composition has a plant extract concentration of ≤ 250 µg/ml, preferably ≤ 150 µg/ml, more preferably ≤ 100 µg/ml.

3. The composition for use according to claim 1 or 2, **characterized in that** the composition has cytoprotective effects, preferably against inflammation related parameters of human cells, more preferably due to the gene expression of IL-1β, IL-6, IL-8 and TNFα.

4. The composition for use according to any one of the preceding claims, **characterized in that** the composition has at least one enriched or chemically modified proanthocyanidin.

5. The composition for use according to any one of the preceding claims, **characterized in that** the composition has at least one enriched and chemically modified proanthocyanidin.

6. The composition for use according to any one of the preceding claims, **characterized in that** said preparation is a preparation for preventing the formation of or for dissolving bacterial coatings, biofilms or plaque.

7. The composition for use according to any one of the preceding claims, **characterized in that** the *Rumex acetosa* plant extract was obtained by a two-stage extraction, having the following steps:
a. extraction in an acetone-water mixture,
b. obtaining the aqueous phase
c. shaking the aqueous phase with ethyl acetate, and
d. obtaining the aqueous phase.

8. The composition for use according to any in one of the preceding claims, **characterized in that** at least one proanthocyanidin is a procyanidin.

9. The composition for use according to any one of the preceding claims, **characterized in that** the composition has at least one oligomeric and a polymeric proanthocyanidin.

10. The composition for use according to any one of the preceding claims, **characterized in that** the composition has at least one tetrameric or higher oligomeric proanthocyanidin.

11. The composition for use according to any one of the preceding claims, **characterized in that** said adhesion of at least one bacterium is provided by a bacterial adhesin.

12. The composition for use according to any one of claims 1 to 11, wherein the composition is a mouth wash, a tincture, an ointment, a toothpaste, a lozenge, a gel, impregnated inserts, rapid-release and dissolving granules, powders or other solid dosage forms, chewing gums or other plastic or elastic masses, impregnated tooth cleaning aids, such as dental floss, toothbrushes or a rinse.

## Patentansprüche

1. Zusammensetzung aufweisend wenigstens eine oligomere oder polymere flavan-3-ol Einheit (Proanthocyanidin) aus einem *Rumex acetosa* Pflanzenextrakt, zur Verwendung in der Prävention oder der Behandlung von Zahnfleischentzündungen, Parodontose, Karies, Geschwüren der oralen Schleimhaut (Aphthen) oder Gingivostomatitis, wobei die Zusammensetzung die Adhäsion wenigstens eines Bakteriums auf der Schleimhaut, Zunge, Gaumen, Zahnfleisch, Zahnfleischtaschen oder Zahnoberfläche, innerhalb einer Expositionszeit, welche 10 Minuten nicht überschreitet, verhindert.

2. Die Zusammensetzung für die Verwendung nach Anspruch 1, **dadurch gekennzeichnet, dass** die Zusammensetzung eine Konzentration an Pflanzenextrakt von ≤ 250 µg/ml, bevorzugt ≤ 150 µg/ml, besonders bevorzugt ≤100 µg/Milliliter aufweist.

3. Die Zusammensetzung für die Verwendung nach Anspruch 1 oder 2, **dadurch gekennzeichnet, dass** die Zusammensetzung zellschützende Effekte aufweist, bevorzugt gegen entzündungszugehörige Parameter humaner Zellen, besonders bevorzugt durch die Genexpression von In-1β, IL-6, IL-8 und TNF α.

4. Die Zusammensetzung für die Verwendung nach einem der vorhergehenden Ansprüche, **dadurch gekennzeichnet, dass** die Zusammensetzung wenigstens ein angereichertes oder chemisch modifiziertes Proanthocyanidin aufweist.

5. Die Zusammensetzung für die Verwendung nach einem der vorhergehenden Ansprüche, **dadurch gekennzeichnet, dass** die Zusammensetzung wenigstens ein angereichertes und chemisch modifiziertes Proanthocyanidin aufweist.

6. Die Zusammensetzung für die Verwendung nach einem der vorhergehenden Ansprüche, **dadurch gekennzeichnet, dass** besagte Zubereitung eine Zubereitung zur Prävention der Bildung oder zum Auflösen bakterieller Filme, Biofilmen oder Plaques ist.

7. Die Zusammensetzung für die Verwendung nach einem der vorhergehenden Ansprüche, **dadurch gekennzeichnet, dass** der *Rumex acetosa* Pflanzenextrakt durch eine zweistufige Extraktion mit folgenden Schritten erhalten wurde:
a. Extraktion in einem Aceton-Wassergemisch,
b. Erhalten der wässrigen Phase
c. Ausschütteln der wässrigen Phase mit Ethylacetat, und
d. Erhalten der wässrigen Phase.

8. Die Zusammensetzung für die Verwendung nach einem der vorhergehenden Ansprüche, **dadurch gekennzeichnet, dass** wenigstens ein Proanthocyanidin ein Procyanidin ist.

9. Die Zusammensetzung für die Verwendung nach einem der vorhergehenden Ansprüche, **dadurch gekennzeichnet, dass** die Zusammensetzung wenigstens ein oligomeres und ein polymeres Proanthocyanidin aufweist.

10. Die Zusammensetzung für die Verwendung nach einem der vorhergehenden Ansprüche, **dadurch gekennzeichnet, dass** die Zusammensetzung wenigstens ein tetrameres oder höher oligomeres Proanthocyanidin aufweist.

11. Die Zusammensetzung für die Verwendung nach einem der vorhergehenden Ansprüche, **dadurch gekennzeichnet, dass** besagte Adhäsion des wenigstens einen Bakteriums durch ein bakterielles Adhäsin erfolgt.

12. Die Zusammensetzung für die Verwendung nach einem der Ansprüche 1-11, wobei die Zusammensetzung ein Mundwasser, eine Tinktur, eine Salbe, eine Zahnpasta, eine Lutschtablette, ein Gel, imprägnierte Einsätze, schnell frei setzendes und sich auflösendes Granulat, Pulver oder andere feste Dosierungsformen, Kaugummis oder andere plastische oder elastische Massen, imprägnierte Zahnreinigungshilfen, wie beispielsweise Zahnseide, Zahnbürsten oder eine Spülung, ist.

## Revendications

1. Composition contenant au moins une unité flavan-3-ol oligomérique ou polymérique (proanthocyanidine) provenant d'un extrait de plante *Rumex acetosa,* pour utilisation dans de la prévention ou du traitement de gingivite, de parodontite, de carie, d'ulcère de la muqueuse orale (aphte), ou de gingivostomatite, dans laquelle la composition empêche l'adhérence d'au moins une bactérie sur la muqueuse, la langue, le palais, la gencive, la poche gingivale ou de la surface de dent dans un temps d'exposition qui ne dépasse pas 10 minutes.

2. Composition pour utilisation selon la revendication 1, **caractérisée en ce que** la composition a une concentration en extrait de plantes de ≤ 250 µg/ml, de préférence ≤ 150 µg/ml, plus de préférence ≤ 100 µg/ml.

3. Composition pour utilisation selon la revendication 1 ou 2, **caractérisée en ce que** la composition a des effets cytoprotecteurs, de préférence contre des paramètres liés à une inflammation de cellules humaines, plus de préférence due à l'expression de gène d'IL-1β, IL-6, IL-8 et TNFα.

4. Composition pour utilisation selon l'une quelconque des revendications précédentes, **caractérisée en ce que** la composition a au moins une proanthocyanidine enrichie ou chimiquement modifiée.

5. Composition pour utilisation selon l'une quelconque des revendications précédentes, **caractérisée en ce que** la composition a au moins une proanthocyanidine enrichie et chimiquement modifiée.

6. Composition pour utilisation selon l'une quelconque des revendications précédentes, **caractérisé en ce que** ladite préparation est une préparation pour empêcher la formation de ou pour dissoudre des revêtements bactériens, de la plaque ou des biofilms.

7. Composition pour utilisation selon l'une quelconque des revendications précédentes, **caractérisée en ce que** l'extrait de plante *Rumex acetosa* a été obtenu par une extraction à deux phases, ayant les étapes suivantes :
a. extraction dans un mélange d'acétone-eau,
b. obtention de la phase aqueuse
c. agitation de la phase aqueuse avec de l'acétate d'éthyle, et
d. obtention de la phase aqueuse.

8. Composition pour utilisation selon l'une quelconque des revendications précédentes, **caractérisée en ce qu'**au moins une proanthocyanidine est une procyanidine.

9. Composition pour utilisation selon l'une quelconque des revendications précédentes, **caractérisée en ce que** la composition a au moins une proanthocyanidine oligomérique et une polymérique.

10. Composition pour utilisation selon l'une quelconque des revendications précédentes, **caractérisée en ce que** la composition a au moins une proanthocyanidine tétramérique ou oligomérique d'ordre supérieur.

11. Composition pour utilisation selon l'une quelconque des revendications précédentes, **caractérisée en ce que** ladite adhérence d'au moins une bactérie est réalisée par une adhésine bactérienne.

12. Composition pour utilisation selon l'une quelconque des revendications 1 à 11, dans laquelle la composition est un bain de bouche, une teinture, un onguent, une pâte dentifrice, une tablette, un gel, des inserts imprégnés, des granules à dissoudre et à libération rapide, des poudres ou d'autres formes de dosage solides, des chewing-gums ou d'autres masses plastiques ou élastiques, des aides au nettoyage dentaire imprégnées, telles que du fil dentaire, des brosses à dents ou un produit de rinçage.
